(19) Europäisches Patentamt
European Patent Office
Office européen des brevets

(11) EP 4 710 858 A1

(12) EUROPEAN PATENT APPLICATION
published in accordance with Art. 153(4) EPC

(43) Date of publication:
**18.03.2026 Bulletin 2026/12**

(21) Application number: **24803318.5**

(22) Date of filing: **08.04.2024**

(51) International Patent Classification (IPC):
***A61B 5/256*** *(2021.01)* ***A61B 5/291*** *(2021.01)*

(52) Cooperative Patent Classification (CPC):
**A61B 5/256; A61B 5/291**

(86) International application number:
**PCT/JP2024/014242**

(87) International publication number:
**WO 2024/232203 (14.11.2024 Gazette 2024/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **09.05.2023 JP 2023077092**

(71) Applicant: **Sumitomo Bakelite Co.Ltd.**
**Shinagawa-ku**
**Tokyo 140-0002 (JP)**

(72) Inventors:
- **KITAZOE, Katsuma**
  **Tokyo 140-0002 (JP)**
- **ONO, Yoshiatsu**
  **Tokyo 140-0002 (JP)**
- **ISHIKAWA, Eiji**
  **Tokyo 140-0002 (JP)**

(74) Representative: **Ter Meer Steinmeister & Partner Patentanwälte mbB**
**Nymphenburger Straße 4**
**80335 München (DE)**

# (54) BRAIN WAVE MEASURING DEVICE AND BRAIN WAVE MEASURING METHOD

(57) Provided is a brain wave measuring device (10) including a support member (20) which is disposed along a head (99) of a subject, an electrode unit (30) which is attached to the support member (20) and acquires a brain wave signal by coming into contact with a scalp of the subject, and an assist member (attachment part (70)) which assists in positioning the support member (20) on the head (99), in which the support member (20) is composed of a ribbon-shaped member or a line-shaped member.

FIG. 1

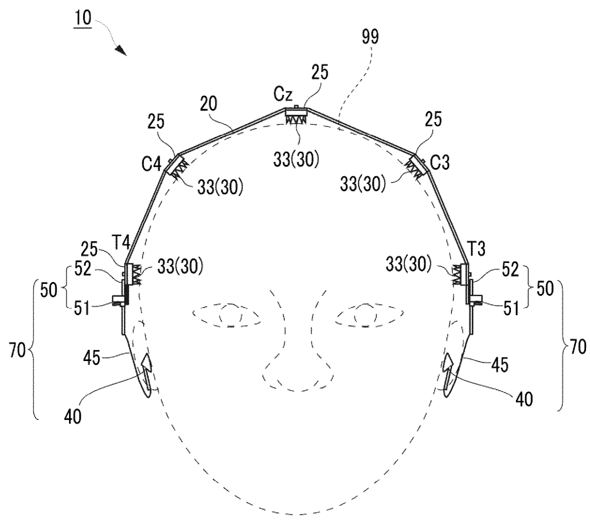


FIG. 2

EP 4 710 858 A1

## Description

TECHNICAL FIELD

The present invention relates to a brain wave measuring device and a brain wave measuring method.

BACKGROUND ART

Various developments have been made on a brain wave measuring device which measures brain waves. As a kind of this technique, for example, a biological signal measuring device has been known, that includes a support which is made of a shape memory material and is mounted on a head of a user, and a vital sensor which is attached to the support and acquires a biological signal of the user (see, for example, Patent Document 1). According to the technique of Patent Document 1, in a case of measuring a biological signal, the support can be easily restored to a shape that matches the body shape of the user in which the shape is stored in advance.

RELATED DOCUMENT

PATENT DOCUMENT

[Patent Document 1] Japanese Patent No. 5900167

SUMMARY OF THE INVENTION

TECHNICAL PROBLEM

Meanwhile, in the measurement of the brain waves, it is important to fit an electrode to a head shape in order to bring the electrode into contact with a scalp. However, the head shape varies widely, and a corresponding technique has been required.

The present invention has been made in view of such circumstances, and an object of the present invention is to provide a brain wave measuring device in a brain wave measurement, particularly a technique for appropriately bringing an electrode into contact with a scalp.

SOLUTION TO PROBLEM

According to the present invention, the following techniques are provided.

1. A brain wave measuring device including:

   a support member which is disposed along a head of a subject;
   an electrode unit which is attached to the support member and acquires a brain wave signal by coming into contact with a scalp of the subject; and
   an assist member which assists in positioning the support member on the head,
   in which the support member is composed of a ribbon-shaped member or a line-shaped member.

2. The brain wave measuring device according to 1.,
in which the support member is composed of a non-stretchable member which has flexibility of being deformable to follow a shape of the head.
3. The brain wave measuring device according to 1. or 2.,
in which, in a case where the support member is composed of the ribbon-shaped member, the support member has a film member which is stretched between adjacent electrode units.
4. The brain wave measuring device according to 3.,
in which a Poisson's ratio of a material constituting the film member is 0.15 to 0.4.
5. The brain wave measuring device according to 3. or 4.,
in which a product of an elastic modulus and a thickness of a material constituting the film member is 0.4 to 9.1 GPa·mm.
6. The brain wave measuring device according to 1. or 2.,
in which, in a case where the support member is composed of the line-shaped member, the line-shaped member is stretched between electrode units.

7. The brain wave measuring device according to 6.,
in which at least two members spaced from each other are provided to extend as the line-shaped member, and the electrode units are provided between the two members.
8. The brain wave measuring device according to 1. to 7.,
in which the electrode unit has a base portion, a plurality of protruding portion provided on the base portion, and an electrode portion provided on the protruding portions and in contact with the head.
9. The brain wave measuring device according to 8.,
in which the protruding portion is an elastic member.
10. The brain wave measuring device according to any one of 1. to 9.,
in which the assist member is provided at an end portion of the support member, is attached to an ear or a jaw, and assists in positioning the support member along a shape of the head.
11. A brain wave measuring method including:

attaching the brain wave measuring device according to any one of 1. to 10. to a head of a subject; and measuring brain waves.

ADVANTAGEOUS EFFECTS OF INVENTION

According to the present invention, it is possible to provide a brain wave measuring device in a brain wave measurement, particularly a technique for appropriately bringing an electrode into contact with a scalp.

BRIEF DESCRIPTION OF THE DRAWINGS

FIG. 1 is a schematic view showing a state in which a brain wave measuring device according to a first embodiment is mounted on a head.
FIG. 2 is a schematic view showing a support member and an attachment part according to the first embodiment.
FIG. 3 is a diagram illustrating, by modeling, a force (electrode pressing force F on the head) acting on the head in a case where the brain wave measuring device is mounted according to the first embodiment.
FIG. 4 is a diagram illustrating a capstan principle (equation) used for the modeling according to the first embodiment.
FIG. 5 is a cross-sectional view of an electrode unit in a state of being mounted on the support member according to the first embodiment.
FIG. 6 is a schematic view showing a state in which a brain wave measuring device according to a second embodiment is mounted on a head.
FIG. 7 is a schematic view showing a support member and an attachment part according to the second embodiment.
FIG. 8 is a cross-sectional view of an electrode unit in a state of being mounted on the support member according to the second embodiment.
FIGS. 9A and 9B are diagrams schematically showing an adjustment part according to the second embodiment.
FIG. 10 is a plan view of a brain wave measuring device according to a third embodiment.
FIG. 11 is a front view of the brain wave measuring device according to the third embodiment.
FIG. 12 is a plan view focusing on an attachment aspect of a support member for two electrode units at electrode positions Cz and C3 according to the third embodiment.
FIG. 13 is a cross-sectional view of the electrode unit in a state of being mounted on the support member according to the third embodiment.
FIG. 14 is a graph showing a theoretical value (calculated value) and a measured value of an electrode pressing force F according to Examples.

DESCRIPTION OF EMBODIMENTS

Hereinafter, embodiments of the present invention will be described with reference to the drawings. In the present embodiment, a brain wave measuring device which has an electrode unit and is mounted on a head of a subject to acquire brain waves, and a brain wave measuring method using the brain wave measuring device will be described.

<First embodiment>

<Schematic structure of brain wave measuring device 10>

FIG. 1 is a schematic view of a state in which a brain wave measuring device 10 is mounted on a head 99 of a person, as viewed from the front. FIG. 2 is a view showing a support member 20 and an attachment part 70, which support the electrode unit 30. In the present embodiment, the brain wave measuring device 10 which measures brain waves at electrode positions Cz, C3, C4, T3, and T4 (international 10-20 method) is described.

The brain wave measuring device 10 is mounted on the head 99 of the person, detects brain waves as a potential fluctuation from a living body, and outputs the detected brain waves to a brain wave display device (not shown). The brain wave display device acquires the brain waves detected by the brain wave measuring device 10, and performs monitor display, data storage, and a well-known brain wave analysis process (measurement process).

The brain wave measuring device 10 has a plurality of electrode units 30 which come into contact with a measurement site (that is, the head 99) of a subject to acquire brain waves as a biological signal, a support member 20 in a long sheet shape (film base material), which supports the electrode units 30 by attachment, and an attachment part 70 which fixes the support member 20 to the head 99. In the present embodiment, the brain wave measuring device 10 is fixed to the head 99 by mounting the attachment part 70 on the ear of the subject.

The support member 20 is configured to be bendable to some extent at least in a thickness direction. In addition, a member (so-called hard member such as a helmet) which is provided in advance with a shape by surrounding the support member 20 is not provided. In a state in which the support member 20 and the like are not mounted on the head 99, the support member 20 and the like can be arranged in a flat state without being applied by an external force; and in a case of being mounted on the head 99, the shape of the support member 20 and the like is mainly changed along the shape of the head 99 by gravity. Since both end portions of the electrode unit 30 may be in a state of being lifted from the head 99 due to influence of head hair or the like, the attachment part 70 is used for supporting the follow-up to the shape of the head 99.

The support member 20 is provided with a female snap button 25 for fixing the electrode unit 30, and a male snap button 35 of the electrode unit 30 is joined to the female snap button 25. In addition, in the support member 20, a position where the female snap buttons 25 are provided in the left-right direction is a bent portion 27 where the support member 20 is bent. In a case where the support member 20 is sufficiently thin, the bent portion 27 is not necessary, but by providing the bent portion 27, the support member 20 between the electrode units 30 can be made linear.

The attachment part 70 has an ear attachment part 40 and an adjustment part 50, and the ear attachment part 40 and the adjustment part 50 are connected to each other by a member (here, a string 45) which does not extend.

In a case where the brain wave measuring device 10 is mounted on the head 99, the position of the electrode unit 30 is generally a vertex of a polygonal shape, and the electrode units 30 are connected to each other by the support member 20. That is, the support member 20 is bent at a portion (bent portion 27) where the electrode unit 30 is exactly attached.

<Electrode pressing force F on head>

Here, features of the present embodiment will be described with reference to FIGS. 3 and 4. FIG. 3 is a diagram illustrating, by modeling, a force (electrode pressing force F on the head) acting on the head 99 in a case where the brain wave measuring device 10 is mounted according to the first embodiment. FIG. 4 is a diagram illustrating a capstan principle (equation) used for the modeling.

As shown in FIG. 3, in a state in which the brain wave measuring device 10 is mounted on the head 99, a polygonal shape is formed with the plurality of electrode units 30 as vertices and the support member 20 connecting the electrode units 30. In this case, assuming that the electrode unit 30 is pressed against the head 99 by a tension of the support member 20, a force (electrode pressing force F) for pressing the electrode unit 30 against the head 99 can be represented by the following expression 1.

$$F = 2 \cdot T_1 \cdot \cos(\theta) \quad \text{... Expression 1}$$

That is, a difference in $\theta$ caused by individual difference in unevenness of the head appears as the difference in the electrode pressing force F.

Furthermore, the minimum curvature of an inscribed circle is obtained from a height and an interval of the electrode units 30. In addition, the maximum curvature is obtained from the capstan principle with the electrode pressing force F required for the electrode unit 30. Since the electrode pressing force F on the head 99 follows the capstan equation, the force is larger on a temporal region than on a parietal region.

Here, it is assumed that the support member 20 (film base material) is in a state of being wound around the head 99. Since there is friction between the head 99 and the support member 20, it is considered that the tension of each electrode unit 30 follows the capstan equation shown in the following expression 2.

$$T_1 = T_2 \cdot e^{(\mu \cdot \varphi)} \quad \text{... Expression 2}$$

$\mu$: friction coefficient
$\varphi$: central angle

A relational expression between a tension T1 applied to the end portion of the support member 20 (film base material) from the expression 1 and expression 2 and a load (electrode pressing force F) applied to the head 99 by the electrode unit 30 can be grasped.

The load value calculated from the above-described theoretical calculation and a load value actually measured will be shown in Examples (see FIG. 6) described later.

<Electrode unit 30>

The electrode unit 30 is attachably and detachably provided only at a portion required for the brain wave measurement. Attachment positions of the electrode units 30 correspond to, for example, the positions of T3, C3, Cz, C4, and T4 in the international 10-20 electrode arrangement method, and are arranged to be symmetrical in front view as shown in FIG. 1.

FIG. 5 shows a cross-sectional view of the electrode unit 30 in a state of being attached to the female snap button 25 of the support member 20. The electrode unit 30 is configured as a button electrode and has a male snap button 35 provided as a connection terminal, and the male snap button 35 is attachably and detachably attached to the female snap button 25 provided at a predetermined position of the support member 20. That is, the female snap button 25 functions as a mounting portion for attaching the electrode unit 30 by the joining structure.

The electrode unit 30 may be composed of a conductive metal as a whole, or may have a configuration in which a conductive member is provided on a surface of a rubber-like elastic member as a base. Hereinafter, the configuration based on the rubber-like elastic member will be exemplified.

The detailed structure and material of the electrode unit 30 will be described later, but the electrode unit 30 according to the present embodiment is an electrode (dry electrode) which does not use a so-called paste for a brain wave electrode in order to ensure conductivity. However, a method of forming a gel on the dry electrode tip (tip of the protrusion portion 32) and immersing the gel in a wet liquid or the like to secure moisture may be adopted. In addition, a method of wetting the dry electrode tip with a conductive auxiliary liquid in which a small amount of an electrolyte such as salt is mixed with a low-viscosity liquid such as a lotion, instead of the paste or a grease, may be adopted. That is, the dry electrode according to the present embodiment is not limited to the complete dry electrode, but is intended not to use an auxiliary agent such as a paste in which significant stains remain.

The electrode unit 30 has a base portion 31 having a columnar shape, a protrusion portion 32 which is provided integrally with one end of the base portion 31 (here, a base portion lower surface 36), a conductive contact portion 33, a signal line portion 34, and a male snap button 35 which is provided at the other end of the base portion 31 (here, a base portion upper surface 37). Hereinafter, the base portion 31 and the protrusion portion 32 will be referred to as an electrode portion main body 39 for convenience.

The electrode portion main body 39 is integrally provided by a rubber-like elastic member. A specific material of the elastic member will be described later. The electrode portion main body 39 (that is, the base portion 31 and the protrusion portion 32) is not limited to the configuration of being integrally provided, and may be configured by assembling those provided separately by an adhesive or a joining structure.

<Shape of base portion 31 and protrusion portion 32>

The base portion 31 has a substantially columnar shape. A plurality of substantially conical protrusion portions 32 which protrude in a downward direction in the drawing are provided on the base portion lower surface 36 on one end side of the base portion 31 (the lower side in the drawing). It is sufficient that the base portion 31 have the columnar shape, and a cross section thereof may have a shape other than a circle, such as a polygonal shape. The shape of the protrusion portion 32 is not limited to the conical shape, and various shapes of a pyramid such as a triangular pyramid, a columnar shape, and the like may be adopted.

The conductive contact portion 33 is provided on at least a distal end side surface of the protrusion portion 32. The conductive contact portion 33 may be provided on the entire surface of the protrusion portion 32. The conductive contact portion 33 is formed in a thin film shape, and in a state in which the conductive contact portion 33 is provided on the protrusion portion 32, it can be regarded as the conductive contact portion 33 having a substantially the same shape as the shape of only the protrusion portion 32.

An outer diameter of the base portion 31 is, for example, 10 mm to 50 mm. A height (thickness) of the base portion

31 is, for example, 0.1 mm to 30 mm. A height of the protrusion portion 32 is, for example, 1 mm to 20 mm. A width (outer diameter of a root portion) of the protrusion portion 32 is, for example, 1 mm to 10 mm.

<Material of electrode portion main body 39 (base portion 31 and protrusion portion 32)>

A material of the electrode portion main body 39 will be described. The electrode portion main body 39 may be made of a rubber-like elastic body as described above. Specifically, the rubber-like elastic body is a rubber or a thermoplastic elastomer (also simply referred to as "elastomer (TPE)"). Examples of the rubber include a silicone rubber. Examples of the thermoplastic elastomer include styrene-based TPE (TPS), olefin-based TPE (TPO), vinyl chloride-based TPE (TPVC), urethane-based TPE (TPU), ester-based TPE (TPEE), and amide-based TPE (TPAE).

In a case where a material of the electrode portion main body 39 is silicone rubber, a rubber hardness A is, for example, 15 or more and 55 or less in a case where a type A durometer hardness of the surface of the electrode portion main body 39 is measured at 37°C in accordance with JIS K 6253 (1997) and is defined as the rubber hardness A.

Here, the above-described silicone rubber-based curable composition will be described.

The above-described silicone rubber can be formed of a cured product of the silicone rubber-based curable composition. A curing step of the silicone rubber-based curable resin composition is performed by heating (primary curing) the composition, for example, at 100°C to 250°C for 1 to 30 minutes and post-baking (secondary curing) the heated composition at 100°C to 200°C for 1 to 4 hours.

An insulating silicone rubber is a silicone rubber not containing a conductive filler, and a conductive silicone rubber is a silicone rubber containing a conductive filler.

The silicone rubber-based curable composition according to the present embodiment may contain a vinyl group-containing organopolysiloxane (A). The vinyl group-containing organopolysiloxane (A) is a polymer including the silicone rubber-based curable composition according to the present embodiment as a main component.

The insulating silicone rubber-based curable composition and the conductive silicone rubber-based curable composition may contain the same kind of vinyl group-containing linear organopolysiloxane. It is sufficient that the same kind of vinyl group-containing linear organopolysiloxane includes at least a vinyl group having the same functional group and is linear, and it may have different vinyl group amounts or molecular weight distributions in the molecule, or different addition amounts thereof.

The insulating silicone rubber-based curable composition and the conductive silicone rubber-based curable composition may contain different kinds of vinyl group-containing organopolysiloxanes.

The above-described vinyl group-containing organopolysiloxane (A) may include a vinyl group-containing linear organopolysiloxane (A1) having a linear structure.

The above-described vinyl group-containing linear organopolysiloxane (A1) has a linear structure and contains a vinyl group, in which the vinyl group functions as a crosslinking point during the curing.

A content of the vinyl group in the vinyl group-containing linear organopolysiloxane (A1) is not particularly limited, and for example, the vinyl group-containing linear organopolysiloxane (A1) has two or more vinyl groups in the molecule, and the content thereof is preferably 15 mol% or less and more preferably 0.01 to 12 mol%. As a result, the amount of the vinyl group in the vinyl group-containing linear organopolysiloxane (A1) can be optimized, and a network between respective components described later can be reliably formed. In the present embodiment, a range represented by "to" includes numerical values of both ends.

In the present specification, the content of the vinyl group represents mol% of a vinyl group-containing siloxane unit with respect to 100 mol% of all units forming the vinyl group-containing linear organopolysiloxane (A1). However, it is assumed that one vinyl group is present for each vinyl group-containing siloxane unit.

In addition, a polymerization degree of the vinyl group-containing linear organopolysiloxane (A1) is not particularly limited, and is, for example, preferably in a range of approximately 1,000 to 10,000 and more preferably in a range of approximately 2,000 to 5,000. The polymerization degree can be obtained as, for example, a number-average polymerization degree (number-average molecular weight) in terms of polystyrene in gel permeation chromatography (GPC) in which chloroform is used as an eluent.

Furthermore, a specific gravity of the vinyl group-containing linear organopolysiloxane (A1) is not particularly limited and is preferably in a range of approximately 0.9 to 1.1.

By using the vinyl group-containing linear organopolysiloxane (A1) having the polymerization degree and the specific gravity within the above-described ranges, heat resistance, flame retardancy, chemical stability, and the like of the obtained silicone rubber can be improved.

It is preferable that the vinyl group-containing linear organopolysiloxane (A1) has a structure represented by Formula (1) .

[Chem. 1]

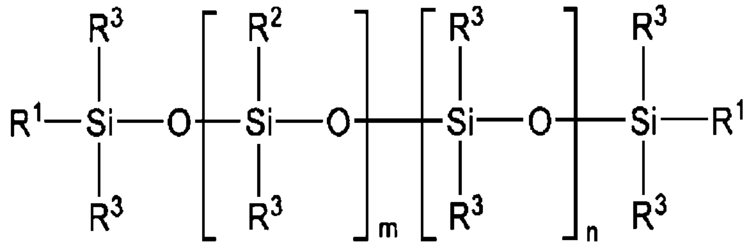

(1)

In Formula (1), $R^1$ represents a substituted or unsubstituted alkyl group, alkenyl group, or aryl group having 1 to 10 carbon atoms, or a hydrocarbon group including a combination thereof. Examples of the alkyl group having 1 to 10 carbon atoms include a methyl group, an ethyl group, and a propyl group; and among these, a methyl group is preferable. Examples of the alkenyl group having 1 to 10 carbon atoms include a vinyl group, an allyl group, and a butenyl group; and among these, a vinyl group is preferable. Examples of the aryl group having 1 to 10 carbon atoms include a phenyl group.

In addition, $R^2$ represents a substituted or unsubstituted alkyl group, alkenyl group, or aryl group having 1 to 10 carbon atoms, or a hydrocarbon group including a combination thereof. Examples of the alkyl group having 1 to 10 carbon atoms include a methyl group, an ethyl group, and a propyl group; and among these, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In addition, $R^3$ represents a substituted or unsubstituted alkyl group or aryl group having 1 to 8 carbon atoms, or a hydrocarbon group including a combination thereof. Examples of the alkyl group having 1 to 8 carbon atoms include a methyl group, an ethyl group, and a propyl group; and among these, a methyl group is preferable. Examples of the aryl group having 1 to 8 carbon atoms include a phenyl group.

Furthermore, in Formula (1), examples of a substituent of $R^1$ and $R^2$ include a methyl group and a vinyl group, and examples of a substituent of $R^3$ include a methyl group.

In Formula (1), a plurality of $R^1$'s may be independent from each other, and may be the same or different from each other. Furthermore, the same can be applied to $R^2$ and $R^3$.

Furthermore, m and n each independently represent the number of repeating units forming the vinyl group-containing linear organopolysiloxane (A1) represented by Formula (1), and m represents an integer of 0 to 2,000 and n represents an integer of 1,000 to 10,000. m preferably represents 0 to 1,000, and n preferably represents 2,000 to 5,000.

In addition, examples of a specific structure of the vinyl group-containing linear organopolysiloxane (A1) represented by Formula (1) include a structure represented by Formula (1-1).

[Chem. 2]

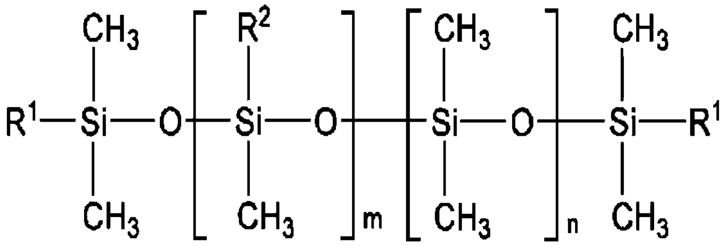

(1-1)

In Formula (1-1), $R^1$ and $R^2$ each independently represent a methyl group or a vinyl group, and at least one thereof represents a vinyl group.

Furthermore, as the vinyl group-containing linear organopolysiloxane (A1), it is preferable to contain a first vinyl group-containing linear organopolysiloxane (A1-1) having two or more vinyl groups in the molecule, in which the content of the vinyl group is 0.4 mol% or less, and a second vinyl group-containing linear organopolysiloxane (A1-2) in which the content of the vinyl group is 0.5 to 15 mol%. By using the first vinyl group-containing linear organopolysiloxane (A1-1) having the general content of the vinyl group and the second vinyl group-containing linear organopolysiloxane (A1-2) having the high content of the vinyl group in combination as raw rubber which is a raw material of the silicone rubber, the vinyl groups can be distributed, and a structure with high crosslinking density in the crosslinked network of the silicone rubber can be more effectively formed. As a result, a tearing strength of the silicone rubber can be more effectively improved.

Specifically, as the vinyl group-containing linear organopolysiloxane (A1), for example, it is preferable to use the first vinyl group-containing linear organopolysiloxane (A1-1) in which, in Formula (1-1) above, two or more units of an unit in which $R^1$ is a vinyl group and/or an unit in which $R^2$ is a vinyl group are included in the molecule and the content of the units is

0.4 mol% or less, and the second vinyl group-containing linear organopolysiloxane (A1-2) in which the content of the unit in which $R^1$ is a vinyl group and/or the unit in which $R^2$ is 0.5 to 15 mol%.

In addition, in the first vinyl group-containing linear organopolysiloxane (A1-1), the content of the vinyl group is preferably 0.01 to 0.2 mol%. In addition, in the second vinyl group-containing linear organopolysiloxane (A1-2), the content of the vinyl group is preferably 0.8 to 12 mol%.

Furthermore, in a case where the first vinyl group-containing linear organopolysiloxane (A1-1) and the second vinyl group-containing linear organopolysiloxane (A1-2) are mixed in combination, a ratio between (A1-1) and (A1-2) is not particularly limited, but a weight ratio (A1-1):(A1-2) is preferably 50:50 to 95:5 and more preferably 80:20 to 90:10.

As each of the first vinyl group-containing linear organopolysiloxane (A1-1) and the second vinyl group-containing linear organopolysiloxane (A1-2), only one kind may be used or two or more kinds may be used in combination.

In addition, the vinyl group-containing organopolysiloxane (A) may include a vinyl group-containing branched organopolysiloxane (A2) having a branched structure.

<<Organohydrogen polysiloxane (B)>>

The silicone rubber-based curable composition according to the present embodiment may contain a crosslinking agent. The crosslinking agent may include an organohydrogen polysiloxane (B) .

The organohydrogen polysiloxane (B) is classified into a linear organohydrogen polysiloxane (B1) having a linear structure and a branched organohydrogen polysiloxane (B2) having a branched structure, and may include either or both of (B1) and (B2).

The insulating silicone rubber-based curable composition and the conductive silicone rubber-based curable composition may contain the same kind of crosslinking agent. It is sufficient that the same kind of crosslinking agent has at least a common structure such as a linear structure or a branched structure, and it may have different molecular weight distributions in the molecule, different functional groups, or different addition amounts thereof.

The insulating silicone rubber-based curable composition and the conductive silicone rubber-based curable composition may further contain different kinds of crosslinking agents.

The linear organohydrogen polysiloxane (B1) is a polymer that has a linear structure and a structure in which hydrogen is directly bonded to Si (=Si-H), and that is obtained by a hydrosilylation reaction of the vinyl group in the vinyl group-containing organopolysiloxane (A) and a vinyl group in a component mixed in the silicone rubber-based curable composition to crosslink the components.

A molecular weight of the linear organohydrogen polysiloxane (B1) is not particularly limited, and for example, a weight-average molecular weight thereof is preferably 20,000 or less and more preferably 1,000 or more and 10,000 or less.

A weight-average molecular weight of the linear organohydrogen polysiloxane (B1) can be measured in terms of polystyrene in gel permeation chromatography (GPC) in which chloroform is used as an eluent.

In addition, it is preferable that the linear organohydrogen polysiloxane (B1) does not have a vinyl group typically. As a result, the crosslinking reaction in the molecule of the linear organohydrogen polysiloxane (B1) can be reliably prevented from progressing.

As such a linear organohydrogen polysiloxane (B1), for example, it is preferable to use an organohydrogen polysiloxane having a structure represented by Formula (2).

[Chem. 3]

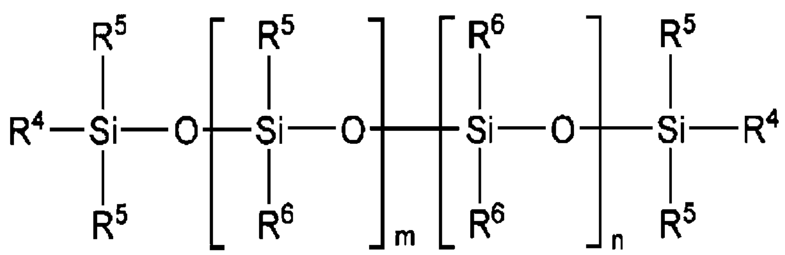

(2)

In Formula (2), $R^4$ represents a substituted or unsubstituted alkyl group, alkenyl group, or aryl group having 1 to 10 carbon atoms, a hydrocarbon group including a combination thereof, or a hydride group. Examples of the alkyl group having 1 to 10 carbon atoms include a methyl group, an ethyl group, and a propyl group; and among these, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In addition, $R^5$ represents a substituted or unsubstituted alkyl group, alkenyl group, or aryl group having 1 to 10 carbon atoms, a hydrocarbon group including a combination thereof, or a hydride group. Examples of the alkyl group having 1 to 10 carbon atoms include a methyl group, an ethyl group, and a propyl group; and among these, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In Formula (2), a plurality of $R^4$'s may be independent from each other, and may be the same or different from each other. The same can be applied to $R^5$. In this case, at least two or more of a plurality of $R^4$'s and $R^5$'s represent a hydride group.

In addition, $R^6$ represents a substituted or unsubstituted alkyl group or aryl group having 1 to 8 carbon atoms, or a hydrocarbon group including a combination thereof. Examples of the alkyl group having 1 to 8 carbon atoms include a methyl group, an ethyl group, and a propyl group; and among these, a methyl group is preferable. Examples of the aryl group having 1 to 8 carbon atoms include a phenyl group. A plurality of $R^6$'s may be independent from each other, and may be the same or different from each other.

Examples of a substituent of $R^4$, $R^5$, and $R^6$ in Formula (2) include a methyl group and a vinyl group, and from the viewpoint of preventing the crosslinking reaction in the molecule, a methyl group is preferable.

Furthermore, m and n each independently represent the number of repeating units forming the linear organohydrogen polysiloxane (B1) represented by Formula (2), and m represents an integer of 2 to 150 and n represents an integer of 2 to 150. It is preferable that m represents an integer of 2 to 100 and n represents an integer of 2 to 100.

As the linear organohydrogen polysiloxane (B1), only one kind may be used alone, or two or more kinds may be used in combination.

Since the branched organohydrogen polysiloxane (B2) has a branched structure, it is a component that largely contributes to a formation of a structure with high crosslinking density in the silicone rubber system by forming a region having a high crosslinking density. In addition, as in the above-described linear organohydrogen polysiloxane (B1), the branched organohydrogen polysiloxane (B2) is a polymer that has a structure in which hydrogen is directly bonded to Si (=Si-H), and that is obtained by a hydrosilylation reaction of the vinyl group in the vinyl group-containing organopolysiloxane (A) and a vinyl group in a component mixed in the silicone rubber-based curable composition to crosslink the components.

In addition, a specific gravity of the branched organohydrogen polysiloxane (B2) is in a range of 0.9 to 0.95.

Furthermore, it is preferable that the branched organohydrogen polysiloxane (B2) does not have a vinyl group typically. As a result, the crosslinking reaction in the molecule of the branched organohydrogen polysiloxane (B2) can be reliably prevented from progressing.

In addition, it is preferable that the branched organohydrogen polysiloxane (B2) is represented by Average Compositional Formula (c) below.

Average compositional formula (c) $$(H_a(R^7)_{3-a}SiO_{1/2})_m(SiO_{4/2})_n$$

(in Formula (c), $R^7$ represents a monovalent organic group, a represents an integer in a range of 1 to 3, m represents the number of $H_a(R^7)_{3-a}SiO_{1/2}$ units, and n represents the number of $SiO_{4/2}$ units)

In Formula (c), $R^7$ represents a monovalent organic group, and preferably represents a substituted or unsubstituted alkyl group or aryl group having 1 to 10 carbon atoms, or a hydrocarbon group including a combination thereof. Examples of the alkyl group having 1 to 10 carbon atoms include a methyl group, an ethyl group, and a propyl group; and among these, a methyl group is preferable. Examples of the aryl group having 1 to 10 carbon atoms include a phenyl group.

In Formula (c), a represents the number of hydride groups (hydrogen atoms directly bonded to Si), which is an integer in a range of 1 to 3 and preferably 1.

In addition, in Formula (c), m represents the number of $H_a(R^7)_{3-a}SiO_{1/2}$ units, and n represents the number of $SiO_{4/2}$ units.

The branched organohydrogen polysiloxane (B2) has a branched structure. The linear organohydrogen polysiloxane (B1) and the branched organohydrogen polysiloxane (B2) are different from each other in that whether the structure is linear or branched. The number (R/Si) of alkyl groups R bonded to Si with respect to the number of Si, which is set to 1, is 1.8 to 2.1 in the linear organohydrogen polysiloxane (B1) and is 0.8 to 1.7 in the branched organohydrogen polysiloxane (B2).

Since the branched organohydrogen polysiloxane (B2) has a branched structure, an amount of residues is 5% or more, for example, after heating to 1,000°C at a temperature increase rate of 10 °C/min in a nitrogen atmosphere. On the other hand, since the linear organohydrogen polysiloxane (B1) is linear, an amount of residues after the heating under the above-described conditions is substantially 0.

In addition, specific examples of the branched organohydrogen polysiloxane (B2) include an organohydrogen polysiloxane having a structure represented by Formula (3).

[Chem. 4]

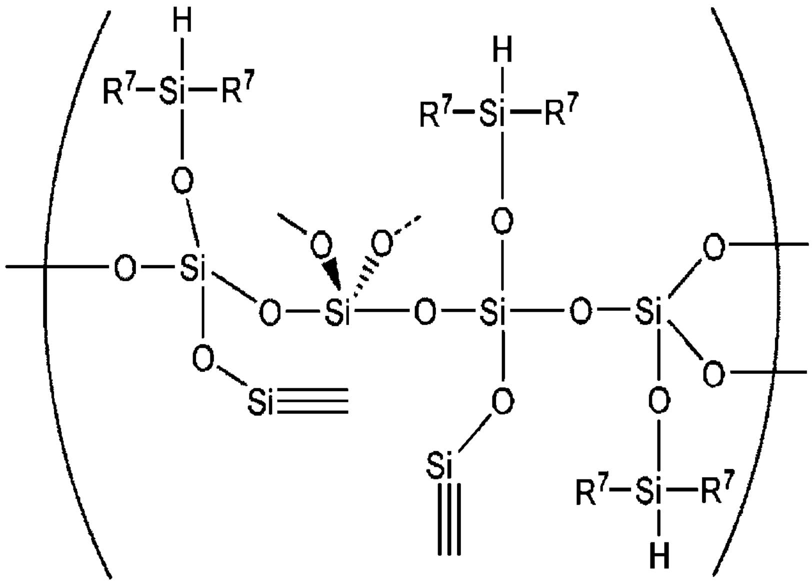

(3)

In Formula (3), $R^7$ represents a substituted or unsubstituted alkyl group or aryl group having 1 to 8 carbon atoms, a hydrocarbon group including a combination thereof, or a hydrogen atom. Examples of the alkyl group having 1 to 8 carbon atoms include a methyl group, an ethyl group, and a propyl group; and among these, a methyl group is preferable. Examples of the aryl group having 1 to 8 carbon atoms include a phenyl group. Examples of a substituent of $R^7$ include a methyl group.

In Formula (3), a plurality of $R^7$'s may be independent from each other, and may be the same or different from each other.

In addition, in Formula (3), "-O-Si=" represents that Si has a branched structure which spreads three-dimensionally.

As the branched organohydrogen polysiloxane (B2), only one kind may be used alone, or two or more kinds may be used in combination.

In addition, in each of the linear organohydrogen polysiloxane (B1) and the branched organohydrogen polysiloxane (B2), an amount of hydrogen atoms directly bonded to Si (hydride groups) is not particularly limited. However, in the silicone rubber-based curable composition, the total amount of hydride groups in the linear organohydrogen polysiloxane (B1) and the branched organohydrogen polysiloxane (B2) is preferably 0.5 to 5 mol and more preferably 1 to 3.5 mol with respect to 1 mol of vinyl groups in the vinyl group-containing linear organopolysiloxane (A1). As a result, a crosslinked network can be reliably formed between the linear organohydrogen polysiloxane (B1) and the branched organohydrogen polysiloxane (B2), and the vinyl group-containing linear organopolysiloxane (A1).

<<Silica particles (C)>>

The silicone rubber-based curable composition according to the present embodiment contains a non-conductive filler. The non-conductive filler may include silica particles (C) as necessary. As a result, hardness or mechanical strength of the elastomer can be improved.

The insulating silicone rubber-based curable composition and the conductive silicone rubber-based curable composition may contain the same kind of non-conductive filler. It is sufficient that the same kind of non-conductive filler may have at least a common constituent material, and the particle diameter, the specific surface area, the surface treatment agent, or the amount added may be different.

The insulating silicone rubber-based curable composition and the conductive silicone rubber-based curable composition may further contain different kinds of silane coupling agents.

The silica particles (C) are not particularly limited, and for example, fumed silica, pyrogenic silica, or precipitated silica is used. These may be used alone or in combination of two or more thereof.

A specific surface area of the silica particles (C), which measured by, for example, a BET method, is, for example, preferably 50 to 400 $m^2/g$ and more preferably 100 to 400 $m^2/g$. In addition, an average primary particle size of the silica particles (C) is, for example, preferably 1 to 100 nm and more preferably approximately 5 to 20 nm.

By using the silica particles (C) having the specific surface area and the average particle size within the above-described range, the hardness or mechanical strength, in particular, the tensile strength of the formed silicone rubber can be improved.

<<Silane coupling agent (D)>>

The silicone rubber-based curable composition according to the present embodiment may contain a silane coupling agent (D).

The silane coupling agent (D) may have a hydrolyzable group. The hydrolyzable group is hydrolyzed into a hydroxyl group by water, this hydroxyl group reacts with a hydroxyl group on a surface of the silica particles (C) in a dehydration synthesis reaction, and as a result, the surface of the silica particles (C) can be modified.

The insulating silicone rubber-based curable composition and the conductive silicone rubber-based curable composition may contain the same kind of silane coupling agent. It is sufficient that the same kind of silane coupling agent has at least a common functional group, and it may have different functional groups in the molecule or different addition amounts thereof.

The insulating silicone rubber-based curable composition and the conductive silicone rubber-based curable composition may further contain different kinds of silane coupling agents.

In addition, the silane coupling agent (D) may include a silane coupling agent having a hydrophobic group. As a result, the hydrophobic group is added to a surface of the silica particles (C), and in the silicone rubber-based curable composition and in the silicone rubber, it is presumed that a cohesion force between the silica particles (C) decreases (cohesion through a hydrogen bond formed by a silanol group decreases), and thus dispersibility of the silica particles (C) in the silicone rubber-based curable composition is improved. Accordingly, an interface between the silica particles (C) and a rubber matrix increases, and a reinforcing effect of the silica particles (C) increases. Furthermore, it is presumed that, when the rubber matrix is deformed, slipperiness of the silica particles (C) in the matrix is improved. By improving the dispersibility and slipperiness of the silica particles (C), a mechanical strength (for example, a tensile strength, a tearing strength, or the like) of the silicone rubber by the silica particles (C) is improved.

Furthermore, the silane coupling agent (D) may include a silane coupling agent having a vinyl group. As a result, the vinyl group is introduced into the surface of the silica particles (C). Therefore, when the silicone rubber-based curable composition is cured, that is, when the vinyl group in the vinyl group-containing organopolysiloxane (A) and the hydride group in the organohydrogen polysiloxane (B) react with each other in a hydrosilylation reaction such that a network (crosslinked structure) is formed, the vinyl group in the silica particles (C) gets involved with the hydrosilylation reaction with the hydride group in the organohydrogen polysiloxane (B). Accordingly, the silica particles (C) are also incorporated into the network. As a result, low hardness and high modulus of the formed silicone rubber can be achieved.

As the silane coupling agent (D), the silane coupling agent having a hydrophobic group and the silane coupling agent having a vinyl group can be used in combination.

Examples of the silane coupling agent (D) include a silane coupling agent represented by Formula (4).

$$Y_n\text{-Si-}(X)_{4-n} \cdots \qquad (4)$$

In Formula (4), n represents an integer of 1 to 3. Y represents any functional group of a hydrophobic group, a hydrophilic group, or a vinyl group, in a case where n represents 1, Y represents a hydrophobic group, and in a case where n represents 2 or 3, at least one of Y's represents a hydrophobic group. X represents a hydrolyzable group.

The hydrophobic group is an alkyl group or aryl group having 1 to 6 carbon atoms, or a hydrocarbon group including a combination thereof. Examples thereof include a methyl group, an ethyl group, a propyl group, and a phenyl group; and among these, a methyl group is particularly preferable.

In addition, examples of the hydrophilic group include a hydroxyl group, a sulfonate group, a carboxyl group, and a carbonyl group; and among these, a hydroxyl group is particularly preferable. The hydrophilic group may be included as a functional group, but from the viewpoint of imparting hydrophobicity to the silane coupling agent (D), it is preferable that the hydrophilic group is not included.

Furthermore, examples of the hydrolyzable group include an alkoxy group such as a methoxy group and an ethoxy group, a chloro group, and a silazane group; and among these, from the viewpoint of high reactivity with the silica particles (C), a silazane group is preferable. A silane coupling agent having a silazane group as the hydrolyzable group has a structure including two structures represented by ($Y_n$-Si-) in Formula (4) above.

Specific examples of the silane coupling agent (D) represented by Formula (4) are as follows.

Examples of the silane coupling agent having a hydrophobic group as the functional group include an alkoxysilane such as methyltrimethoxysilane, dimethyldimethoxysilane, phenyltrimethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, and decyltrimethoxysilane; a chlorosilane such as methyltrichlorosilane, dimethyldichlorosilane, trimethylchlorosilane, and phenyltrichlorosilane; and hexamethyldisilazane. Among these, a silane coupling agent having

a trimethylsilyl group, which includes one or more selected from the group consisting of hexamethyldisilazane, trimethylchlorosilane, trimethylmethoxysilane, and trimethylethoxysilane, is preferable.

Examples of the silane coupling agent having a vinyl group as the functional group include an alkoxysilane such as methacryloxypropyltriethoxysilane, methacryloxypropyltrimethoxysilane, methacryloxypropylmethyldiethoxysilane, methacryloxypropylmethyldimethoxysilane, vinyltriethoxysilane, vinyltrimethoxysilane, and vinylmethyldimethoxysilane; a chlorosilane such as vinyltrichlorosilane and vinylmethyldichlorosilane; and divinyltetramethyldisilazane. Among these, a silane coupling agent having a vinyl group-containing organosilyl group, which includes one or more selected from the group consisting of methacryloxypropyltriethoxysilane, methacryloxypropyltrimethoxysilane, methacryloxypropylmethyldiethoxysilane, methacryloxypropylmethyldimethoxysilane, divinyltetramethyldisilazane, vinyltriethoxysilane, vinyltrimethoxysilane, and vinylmethyldimethoxysilane, is preferable.

In addition, in a case where the silane coupling agent (D) includes two kinds including the silane coupling agent having a trimethylsilyl group and the silane coupling agent having a vinyl group-containing organosilyl group, it is preferable that hexamethyldisilazane is included as the silane coupling agent having a hydrophobic group and divinyltetramethyldisilazane is included as the silane coupling agent having a vinyl group.

In a case where a silane coupling agent (D1) having a trimethylsilyl group and a silane coupling agent (D2) having a vinyl group-containing organosilyl group are used in combination, a ratio between (D1) and (D2) is not particularly limited, but a weight ratio (D1):(D2) is 1:0.001 to 1:0.35, preferably 1:0.01 to 1:0.20 and more preferably 1:0.03 to 1:0.15. By setting such a numerical range, desired physical properties of the silicone rubber can be obtained. Specifically, a balance between the dispersibility of silica in the rubber and the crosslinkability of the rubber can be achieved.

In the present embodiment, a lower limit value of a content of the silane coupling agent (D) is preferably 1 mass% or more, more preferably 3 mass% or more, and still more preferably 5 mass% or more with respect to 100 parts by weight of the total amount of the vinyl group-containing organopolysiloxane (A). In addition, an upper limit value of the content of the silane coupling agent (D) is preferably 100 mass% or less, more preferably 80 mass% or less, and still more preferably 40 mass% or less with respect to 100 parts by weight of the total amount of the vinyl group-containing organopolysiloxane (A).

By adjusting the content of the silane coupling agent (D) to be the above-described lower limit value or more, adhesiveness between a cylindrical portion containing an elastomer and a conductive resin layer can be improved. In addition, the improvement of the mechanical strength of the silicone rubber can be promoted. In addition, by adjusting the content of the silane coupling agent (D) to be the above-described upper limit value or less, the silicone rubber can have appropriate mechanical properties.

<<Platinum or platinum compound (E)>>

The silicone rubber-based curable composition according to the present embodiment may contain a catalyst. The catalyst may include a platinum or a platinum compound (E). The platinum or platinum compound (E) is a catalyst component which functions as a catalyst during the curing. The addition amount of the platinum or platinum compound (E) is the amount of the catalyst.

The insulating silicone rubber-based curable composition and the conductive silicone rubber-based curable composition may contain the same kind of catalyst. It is sufficient that the same kind of catalyst has at least a common constituent material, and it may have different compositions in the catalyst or different addition amounts thereof.

The insulating silicone rubber-based curable composition and the conductive silicone rubber-based curable composition may further contain different kinds of catalysts.

As the platinum or platinum compound (E), a well-known compound can be used, and examples thereof include platinum black, silica or carbon black on which platinum is supported, chloroplatinic acid or an alcohol solution of chloroplatinic acid, a complex salt of chloroplatinic acid and olefin, and a complex salt of chloroplatinic acid and vinylsilxoane.

As the platinum or platinum compound (E), only one kind may be used alone or two or more kinds may be used in combination.

In the present embodiment, a content of the platinum or platinum compound (E) in the silicone rubber-based curable composition refers to the amount of the catalyst and can be appropriately set. Specifically, a content of platinum-group metal in units of weight is 0.01 to 1000 ppm, preferably 0.1 to 500 ppm, with respect to 100 parts by weight of the total amount of the vinyl group-containing organopolysiloxane (A), the silica particles (C), and the silane coupling agent (D).

By adjusting the content of the platinum or platinum compound (E) to be the above-described lower limit value or more, the silicone rubber-based curable composition can be cured at an appropriate rate. In addition, by adjusting the content of the platinum or platinum compound (E) to be the above-described upper limit value or less, a reduction in manufacturing costs can be promoted.

<<Water (F)>>

In addition, the silicone rubber-based curable composition according to the present embodiment may contain water (F) in addition to the above-described components (A) to (E).

The water (F) is a component which functions as a dispersion medium for dispersing the respective components in the silicone rubber-based curable composition and contributes to the reaction between the silica particles (C) and the silane coupling agent (D). Therefore, in the silicone rubber, the silica particles (C) and the silane coupling agent (D) can be more reliably linked to each other, and uniform properties can be exhibited as a whole.

(Other components)

Furthermore, the silicone rubber-based curable composition according to the present embodiment may further contain other components in addition to the above-described components (A) to (F). Examples of the other components include an inorganic filler other than the silica particles (C), such as diatomaceous earth, iron oxide, zinc oxide, titanium oxide, barium oxide, magnesium oxide, cerium oxide, calcium carbonate, magnesium carbonate, zinc carbonate, glass wool, and mica; and an additive such as a reaction inhibitor, a dispersant, a pigment, a dye, an antistatic agent, an antioxidant, a flame retardant, and a thermal conductivity enhancing agent.

The conductive solution (conductive silicone rubber composition) according to the present embodiment contains the above-described conductive filler and a solvent, in addition to the above-described silicone rubber-based curable composition not containing the conductive filler.

As the above-described solvent, various well-known solvents can be used, and for example, a high boiling point solvent can be contained. These may be used alone or in combination of two or more thereof.

Examples of the solvent include an aliphatic hydrocarbon such as pentane, hexane, cyclohexane, heptane, methylcyclohexane, ethylcyclohexane, octane, decane, dodecane, and tetradecane; an aromatic hydrocarbon such as benzene, toluene, ethylbenzene, xylene, trifluoromethylbenzene, and benzotrifluoride; an ether such as diethyl ether, diisopropyl ether, dibutyl ether, cyclopentyl methyl ether, cyclopentyl ethyl ether, ethylene glycol dimethyl ether, ethylene glycol diethyl ether, diethylene glycol dimethyl ether, 1,4-dioxane, 1,3-dioxane, and tetrahydrofuran; a haloalkane such as dichloromethane, chloroform, 1,1-dichloroethane, 1,2-dichloroethane, 1,1,1-trichloroethane, and 1,1,2-trichloroethane; a carboxylic acid amid such as N,N-dimethylformamide and N,N-dimethylacetamide; and a sulfoxide such as dimethyl sulfoxide and diethyl sulfoxide. These may be used alone or in combination of two or more thereof.

By adjusting the solid content in the solution, the above-described conductive solution can have an appropriate viscosity for various coating methods such as spray coating and dip coating.

In addition, in a case where the above-described conductive solution contains the above-described conductive filler and the above-described silica particles (C), a lower limit value of a content of the silica particles (C) in the electrode portion main body 39 is, for example, 1 mass% or more, preferably 3 mass% or more and more preferably 5 mass% or more, with respect to 100 mass% of the total amount of the silica particles (C) and the conductive filler. As a result, the mechanical strength of the electrode portion main body 39 can be improved. On the other hand, an upper limit value of the above-described content of the silica particles (C) in the electrode portion main body 39 is, for example, 20 mass% or less, preferably 15 mass% or less and more preferably 10 mass% or less, with respect to 100 mass% of the total amount of the silica particles (C) and the conductive filler. As a result, a balance between the conductivity and the mechanical strength or flexibility in the electrode portion main body 39 can be achieved.

By optionally heating and drying the conductive solution, the conductive silicone rubber is obtained.

The conductive silicone rubber may be configured not to contain a silicone oil. As a result, a decrease in conductivity due to bleeding out the silicone oil to a surface of the electrode portion main body 39 can be suppressed.

<Material of conductive contact portion 33>

A conductive member of the conductive contact portion 33 is, for example, a paste containing a highly conductive metal (so-called conductive paste). The highly conductive metal includes one or more selected from the group consisting of copper, silver, gold, nickel, tin, lead, zinc, bismuth, antimony, and an alloy thereof. In particular, from the viewpoint of availability and conductivity, silver, silver chloride, or copper is preferable.

In a case where the conductive contact portion 33 is formed with the paste containing a highly conductive metal, an apex portion of the protrusion portion 32 made of the rubber-like elastic body is dipped (dip-coated) in a paste-like conductive solution containing the highly conductive metal. As a result, the conductive contact portion 33 is formed on the surface of the protrusion portion 32.

The conductive contact portion 33 as a conductive resin layer may be formed by applying the conductive solution containing the conductive filler and the solvent onto the protrusion portion 32. In this case, the solvent is made of the same material (silicone rubber) as the material of the protrusion portion 32, so that the adhesiveness of the conductive contact

portion 33 (conductive resin layer) can be enhanced.

By optionally heating and drying the conductive solution, the conductive silicone rubber is obtained.

The conductive silicone rubber may be configured not to contain a silicone oil. As a result, a decrease in conductivity due to bleeding out the silicone oil to a surface of the conductive contact portion 33 can be suppressed.

As a result, it is possible to improve the hair separating performance in a case where the brain wave measuring device 1 is mounted on the head 99. In addition, it is possible to sufficiently secure the contact area of the conductive contact portion 33 in a case where the brain wave measuring device 1 is mounted.

<Structure of signal line portion 34>

The electrode unit 30 is provided with a signal line portion 34 as a signal path connected to the conductive contact portion 33. Various wiring structures can be adopted for the signal line portion 34 as long as the signal line portion 34 is in a state of being conducted through the base portion 31 and the protrusion portion 32. Here, the signal line portion 34 is provided to pass through the inside of the protrusion portion 32 and the base portion 31 from the conductive contact portion 33 at the tip of the protrusion portion 32 and to be exposed on the base portion upper surface 37. A portion (here, an end portion 34a) of the signal line portion 34 which protrudes from the base portion upper surface 37 is interposed between the male snap button 35 (more specifically, a disk portion 35a described later) and the base portion upper surface 37, and conduction with the male snap button 35 is secured.

The lower-side tip of the signal line portion 34 may have either of a protruding structure, a substantially coplanar structure, or a buried structure with respect to a distal end portion of the protrusion portion 32 or the vicinity thereof, that is, the region where the conductive contact portion 33 is formed. From the viewpoint of connection stability with the conductive contact portion 33, a protruding structure may be used. A protrusion portion at the tip of the signal line portion 34 is partially or entirely covered with the conductive contact portion 33. As the protruding structure of the tip of the signal line portion 34, a structure without folding, a structure with folding, or a structure in which the signal line portion 34 is wound around the surface of the distal end portion of the protrusion portion 32 may be adopted.

As another wiring structure of the signal line portion 34, a structure provided on the surfaces of the protrusion portion 32 and the base portion 31 may be used, or a wiring structure in which a part is provided on the inside and a part is provided on the surface may be used. That is, a signal detected by the conductive contact portion 33 may be finally transmitted to the male snap button 35.

<Material of signal line portion 34>

As the signal line portion 34, a well-known material can be used, and for example, the signal line portion 34 can be formed of a conductive fiber. As the conductive fiber, one or more selected from the group consisting of metal fiber, metal-coated fiber, carbon fiber, conductive polymer fiber, conductive polymer-coated fiber, and conductive paste-coated fiber can be used. These may be used alone or in combination of two or more thereof.

A metal material of the metal fiber and the metal-coated fiber described above is not particularly limited as long as it has conductivity, and examples thereof include copper, silver, gold, nickel, tin, lead, zinc, bismuth, antimony, stainless steel, aluminum, and an alloy thereof. These may be used alone or in combination of two or more thereof. Among these, from the viewpoint of conductivity, silver can be used. In addition, it is preferable that the metal material does not include metal such as chromium, which imposes burden on the environment.

The fiber material of the metal-coated fiber, the conductive polymer-coated fiber, or the conductive paste-coated fiber described above is not particularly limited and may be any one of synthetic fiber, semisynthetic fiber, or natural fiber. Among these, for example, polyester, nylon, polyurethane, silk, or cotton is preferably used. These may be used alone or in combination of two or more thereof.

Examples of the above-described carbon fiber include a PAN-based carbon fiber and a pitch-based carbon fiber.

As a conductive polymer material of the conductive polymer fiber and the conductive polymer-coated fiber described above, for example, polythiophene, polypyrrole, polyaniline, polyacetylene, polyphenylene vinylene, polynaphthalene, a mixture of the conductive polymer and the binder resin, for example, derivatives thereof, or an aqueous solution of the conductive polymer PEDOT-PSS ((3,4-ethylenedioxythiophene)-poly(styrene sulfonate)) is used.

A resin material in the conductive paste of the conductive paste-coated fiber described above is not particularly limited and preferably has elasticity. For example, the resin material includes one or more selected from the group consisting of silicone rubber, urethane rubber, fluorine rubber, nitrile rubber, acrylic rubber, styrene rubber, chloroprene rubber, and ethylene propylene rubber. These may be used alone or in combination of two or more thereof.

A conductive filler in the conductive paste of the conductive paste-coated fiber described above is not particularly limited, and a well-known conductive material may be used. For example, the conductive filler may include one or more selected from the group consisting of metal particles, metal fiber, metal-coated fiber, carbon black, acetylene black, graphite, carbon fiber, carbon nanotube, a conductive polymer, conductive polymer-coated fiber, and metal nanowire.

A metal forming the above-described conductive filler is not particularly limited. For example, the metal may include at least one or two or more among copper, silver, gold, nickel, tin, lead, zinc, bismuth, antimony, silver or silver chloride, and alloys thereof. Among these, from the viewpoint of high conductivity or high availability, silver or copper is preferable.

The signal line portion 34 may be formed of twisted yarn obtained by twisting a plurality of linear conductive fibers. As a result, disconnection of the signal line portion 34 during deformation can be suppressed.

In the present embodiment, the coating of the conductive fiber includes not only that the outer surface of the fiber material is covered with the conductive fiber, but also that gaps between fibers in twisted yarn obtained by twisting single fibers are impregnated with metal, the conductive polymer, or the conductive paste such that each of the single fibers forming the twisted yarn is coated with the conductive fiber.

A tensile elongation at break of the signal line portion 34 is, for example, 1% or more and 50% or less, preferably 1.5% or more and 45%. In such a numerical range, excessive deformation of the protrusion portion 32 can be suppressed while suppressing break during deformation.

<Female snap button 25 and male snap button 35>

The male snap button 35 is made of, for example, a highly conductive metal, and has the disk-shaped disk portion 35a and a convex button-shaped button portion 35b which extends from the center of the upper surface of the disk portion 35a. As the highly conductive metal, for example, stainless steel, a copper alloy, an aluminum alloy, brass, or the like can be used.

The disk portion 35a is attached to the base portion upper surface 37 of the base portion 31 with a conductive adhesive or the like. In this case, as described above, the end portion 34a of the signal line portion 34 is interposed between the disk portion 35a and the base portion upper surface 37, and thus the conduction with the male snap button 35 is secured.

The button portion 35b is attached to be joined to the female snap button 25 provided in the support member 20.

As in the male snap button 35, the female snap button 25 is made of the highly conductive metal, and outputs the brain waves acquired by the electrode unit 30 to a brain wave display device or the like through a predetermined wiring structure (not shown).

<Support member 20>

The support member 20 has a ribbon-shaped member. More specifically, the support member 20 is configured to have a long strip-shaped film base material. The support member 20 may be formed of one type of film base material, or may be a composite member formed of a plurality of film base materials. In any case, the support member 20 is configured to have sufficient strength for attaching the electrode unit 30. That is, the support member 20 has sufficient strength not to be damaged in a case where the female snap button 25 is provided to attach the electrode unit 30. In addition, the plurality of electrode units 30 have non-stretchable physical properties (non-stretchable property) from the viewpoint of pressing the plurality of electrode units 30 against the head 99 with an appropriate pressure, that is, applying a constant tension. The non-stretchable property refers to, for example, that a Poisson's ratio described later is within a predetermined range.

A vertical width (length in a depth direction) of the support member 20 depends on the size of the electrode unit 30 to be mounted, but for example, can be 5 mm to 50 mm.

A lateral width (length in a left-right direction) of the support member 20 depends on the size of the head 99 and the electrode positions, but for example, can be 200 mm to 400 mm.

A thickness of the support member 20 depends on the material, but for example, can be 0.01 mm to 5 mm. By setting the thickness of the support member 20 to be within the above-described range, the electrode unit 30 can be pressed with a constant tension. The support member 20 may be curved to the extent that the tension does not substantially change, depending on the shape of the head 99 and the state of the hair.

As a material of the support member 20 (film member), for example, a resin member, a metal member, or a glass film can be used. Examples of the resin member include polyimide resin-based resin films such as a polyimide resin film, a polyetherimide resin film, and a polyamideimide resin film; polyamide resin-based films such as a polyamide resin film; polyester resin-based films such as a polyester resin film; polyethylene terephthalate (PET)-based resin films; and polystyrene (PS)-based resin films. Among the above, from the viewpoint of improving bending resistance, elastic modulus, and heat resistance, a polyimide resin-based film is particularly preferably used. As the metal member, for example, an aluminum foil or a copper foil can be used.

<Physical property value and the like of support member 20>

Physical property values (Poisson's ratio, Young's modulus, maximum thickness, Young's modulus × thickness)

of the support member 20 are defined, for example, as follows.

(Poisson's ratio)

A Poisson's ratio of the support member 20 is 0.15 to 0.4. The lower limit of the Poisson's ratio is preferably 0.2 or more, and more preferably 0.25 or more. The upper limit of the Poisson's ratio is preferably 0.38 or less, and more preferably 0.35 or less.

By setting the Poisson's ratio of the support member 20 to be within the above-described range, the support member 20 is less likely to be deformed. That is, in a case where the brain wave measuring device 10 is mounted on the head 99, a force acts in a direction in which the support member 20 is extended in a state in which the electrode unit 30 is pressed against the head 99. That is, a tension acts. In this case, in a case where the support member 20 is an elastic member such as rubber, the support member 20 may be improperly stretched, the tension of the electrode unit 30 on the head 99 may change, and the pressing force may be non-uniform. As a result, signal quality of the obtained brain waves may be deteriorated. However, by setting the Poisson's ratio of the support member 20 to be within the above-described range, the force with which the electrode unit 30 presses the head 99 can be controlled to be in a certain range, and thus the brain wave measurement can be performed stably.

(Young's modulus)

A Young's modulus (elastic modulus) of the support member 20 is 0.4 GPa to 150 GPa. The lower limit value of the Young's modulus is preferably 3 GPa or more, and more preferably 5 GPa or more. The upper limit value thereof is preferably 140 GPa or less and more preferably 135 GPa or less.

By setting the Young's modulus of the support member 20 to be within the above-described range, the strength of the support member 20 can be maintained, and thus deformation can be prevented.

(Young's modulus × thickness)

A product of the Young's modulus (elastic modulus) and a thickness of a material forming the film member is 0.4 to 9.1 GPa·mm.

Even in a case where the material is easily deformable (that is, the material has a small Young's modulus), as the thickness is equal to or more than a certain degree, the force with which the electrode unit 30 presses the head 99 can be controlled without substantially deforming the material. In addition, in a case of a hard material (that is, a material having a large Young's modulus), in a case where the thickness is not reduced to some extent, the characteristic of causing the brain wave measuring device 10 to follow the shape of the head 99 is significantly deteriorated. Therefore, by setting the product of the Young's modulus (elastic modulus) and the thickness within the above-described range, the force with which the electrode unit 30 attached to the support member 20 presses the head 99 can be controlled within a certain range, and thus stable measurement of the brain waves can be achieved.

<Attachment part 70>

The attachment part 70 is attached to both ends of the support member 20 in the longitudinal direction (both ends in the lateral direction in FIGS. 1 and 2), and is stretched between a part (here, an ear) different from the measurement part of the subject and the support member 20 to press the electrode unit 30 against the head 99 with the predetermined electrode pressing force F.

Specifically, the attachment part 70 includes an adjustment part 50, an ear attachment part 40, and a string 45 which connects the adjustment part 50 and the ear attachment part 40.

The ear attachment part 40 is mounted on the ear of the subject. In the present embodiment, the ear attachment part 40 is mounted so as to be wound from below the ear.

The adjustment part 50 is attached to each of both ends of the support member 20. The adjustment part 50 includes a plate-shaped member 52 attached to the support member 20 and a locking portion 51 for fixing a length of the plate-shaped member 52.

The plate-shaped member 52 has a long strip shape in which a plurality of teeth are arranged in a row. The locking portion 51 is provided with an opening in which a claw is formed, and the plate-shaped member 52 is inserted into the opening and locked at a desired position. In addition, the locking portion 51 is provided with a release portion which releases the locked state in conjunction with the claw. A material of the adjustment part 50 is not particularly limited, and various plastics can be used. From the viewpoint of processability, cost, and the like, Nylon 66 can be preferably used.

As described above, according to the present embodiment, in the brain wave measuring device 10, the electrode unit 30 is fixed to the support member 20 composed of the film base material, and a polygonal shape is formed with the

electrode unit 30 attached only to the required portion as the vertex. Therefore, followability of the electrode unit 30 to the head shape can be improved. In addition, since the mounting state of the brain wave measuring device 10 is modeled by the capstan equation, the electrode pressing force F by the electrode unit 30 can be appropriately grasped.

<Second embodiment>

A second embodiment will be described with reference to FIGS. 6 to 9A and 9B. The present embodiment is different from the first embodiment in that (1) a mounting structure of the electrode unit 30 on the support member 20 is a screw joining structure, (2) an electrode attachment portion 160 is provided instead of the female snap button 25 as a structure for attaching the electrode unit 30 in accordance with the adoption of the screw joining structure, and (3) a structure and an installation position of the adjustment part 150 are different. Hereinafter, the points different from the first embodiment will be mainly described, and the same configurations will be denoted by the same reference numerals as appropriate and the description thereof will not be repeated.

FIG. 6 is a schematic view of a state in which a brain wave measuring device 110 is mounted on a head 99 of a person, as viewed from the front. FIG. 7 is a plan view showing the brain wave measuring device 110 in a state in which the brain wave measuring device 110 is not mounted on the head 99. FIG. 8 is a cross-sectional view showing a state in which the electrode unit 30 is attached to the electrode attachment portion 160 of the support member 20.

<Electrode attachment portion 160>

The support member 20 is provided with the electrode attachment portion 160 for attaching the electrode unit 30. The electrode attachment portion 160 has an attachment portion main body 161 which is circular in top view and a female screw 162 which is provided at the center of the attachment portion main body 161.

An outer diameter of the attachment portion main body 161 is set to be substantially the same as the outer diameter of the electrode unit 30. A circuit unit 163 (pre-amplifier) which is formed of a hard insulating substrate and performs primary amplification on the brain waves acquired by the electrode unit 30 is mounted on the upper surface of the attachment portion main body 161. An upper surface of the attachment portion main body 161 is covered with an insulating cover member as necessary.

The female screw 162 made of a conductive member is attached to the center of the attachment portion main body 161. A male screw connection terminal 135 (protruding portion 135b) of the electrode unit 30 is screw-joined to the female screw 162.

<Male screw connection terminal 135>

On the upper surface of the electrode unit 30, instead of the male snap button 35 according to the first embodiment, the male screw connection terminal 135 made of a conductive member is provided

The male screw connection terminal 135 has a disk-shaped disk portion 135a and a protruding portion 135b which extends from a center of an upper surface of the disk portion 135a. The protruding portion 135b is formed in a cylindrical shape, and a male screw is formed on a peripheral surface thereof. The female screw 162 is connected to the circuit unit 163.

The electrode unit 30 is attached to the support member 20 by screw-joining the female screw 162 and the male screw connection terminal 135 (that is, the protruding portion 135b) to each other. The brain wave signal acquired by the electrode unit 30 is transmitted from the male screw connection terminal 135 to the circuit unit 163 through the female screw 162. The brain wave signal which has been subjected to primary amplification by the circuit unit 163 is output to a brain wave display device or the like through a signal path (not illustrated).

By adopting the screw joining as the attachment aspect of the electrode unit 30 and the support member 20, the attachment portion can be stabilized and generation of noise can be suppressed.

<Adjustment part 150>

FIGS. 9A and 9B show the adjustment part 150. FIG. 9A is a side view, and FIG. 9B is a plan view.

As shown in FIG. 6, the adjustment part 150 is sewn to the support member 20 between the electrode positions C3 and T3 and between the electrode positions C4 and T4, respectively.

The adjustment part 150 includes a plate-shaped member 152 which is attached to the support member 20, and a locking portion 151 which slides the plate-shaped member 152 and fixes the plate-shaped member 152 at a desired position. The locking portion 151 is provided with a string attachment portion 154 to which the string 45 is attached.

Convex string guide portions 126 are provided at both ends of the support member 20 in the longitudinal direction. The string guide portion 126 is provided with an opening communicating in the left-right direction, and the string 45 is

inserted through the opening. As a result, the string 45 always passes through both end portions of a base material 21, and thus the mounting state of the brain wave measuring device 10 can be made appropriate.

The plate-shaped member 152 is a rail-like long strip-shaped member in which a plurality of protruding portions 152a are arranged in a row. The locking portion 151 is slidably joined to a rail formed by the protruding portions 152a. The locking portion 151 has a locking mechanism 156 holding in a non-slidable manner. The non-slidable state is released by a predetermined operation (for example, an operation of pushing in the horizontal direction) with respect to the lock mechanism 156.

By sliding the locking portion 151 on the plate-shaped member 152, a distance between the ear attachment part 40 attached to the string 45 and the support member 20 can be adjusted.

<Third embodiment>

A third embodiment will be described with reference to FIGS. 10 to 13. The present embodiment is different from the first and second embodiments in that (1) a support member 220 is a linear member 221 and (2) the linear member 221 is configured to be detachable. Hereinafter, the points different from the first and second embodiments will be mainly described.

FIG. 10 is a plan view of a brain wave measuring device 210 according to the present embodiment. FIG. 11 is a front view of the brain wave measuring device 210. FIGS. 10 and 11 show the brain wave measuring device 210 in a state in which the brain wave measuring device 210 is not mounted on the head 99. FIG. 12 is a plan view focusing on an attachment aspect of the support member 220 for two electrode units 30 at electrode positions Cz and C3. FIG. 13 is a cross-sectional view of the electrode unit 30 in a state of being attached to an electrode attachment portion 260.

The electrode unit 30 is attached to the electrode attachment portion 260 having the same configuration as the electrode attachment portion 160 shown in the second embodiment, and is supported by the support member 220. The support member 220 is provided to have two parallel linear members 221 which are provided between adjacent electrode units 30 (that is, the electrode attachment portions 260). In addition, an attachment part 270 is attached to each of end portion sides of the electrode attachment portions 260 (electrode positions T3 and T4) at right and left end portions.

<Electrode attachment portion 260>

As in the second embodiment, the electrode attachment portion 260 has an attachment portion main body 261 which is circular in top view, a female screw 262 which is provided at the center of the attachment portion main body 261, and a circuit unit 163 which performs primary amplification on the acquired brain waves.

<Connector 225>

The electrode attachment portion 260 has a plurality of connectors 225 for attaching the support member 220 (linear member 221).

On the upper surface of three of the electrode attachment portions 260 in the center (that is, electrode positions Cz, C3, and C4), two connectors 265 are provided on the right side in the drawing at the same position in the left-right direction on the front side and the back side, and two connectors 265 are provided on the left side in the drawing at the same position in the left-right direction on the front side and the back side.

The two connectors 265 on the right side each have an opening facing the right side, and are insertable into and removable from the connector 225 provided on the linear member 221. The two connectors 265 on the left side each have an opening facing the left side, and are insertable into and removable from the connector 225 provided on the linear member 221.

On the upper surface of the electrode attachment portion 260 at the right end (electrode position T3), two connectors 265 are arranged on the left side in the drawing, and an attachment part 270 (adjustment part 250) is provided near the center on the right side. The two connectors 265 each have an opening facing the left side, and are insertable into and removable from the connector 225 provided on the linear member 221.

On the upper surface of the electrode attachment portion 260 at the left end (electrode position T4), two connectors 265 are arranged on the right side in the drawing, and an attachment part 270 (adjustment part 250) is provided near the center on the left side. The two connectors 265 each have an opening facing the right side, and are insertable into and removable from the connector 225 provided on the linear member 221.

<Attachment part 270>

As in the attachment part 70 according to the second embodiment, the attachment part 270 has an ear attachment part 240, an adjustment part 250, and a string 245. In the present embodiment, an attachment position of

the adjustment part 250 is the electrode attachment portion 260.

The adjustment part 250 has a locking portion 251 attached to the electrode attachment portion 260 and a plate-shaped member 252 fixed in position by the locking portion 251. The string 245 is attached to the plate-shaped member 252.

By sliding the plate-shaped member 252, a distance between the ear attachment part 240 attached to the string 245 and the electrode attachment portion 260 can be adjusted.

<Support member 220>

The support member 220 has a linear member 221 and connectors 225 provided at both ends of the linear member 221.

The linear member 221 refers to a line-shaped member such as a string and a wire. As the linear member 221, for example, an electric wire cable (a multi-core cable, a flat cable, or the like) can be used. A length of the linear member 221 is set according to the brain wave measurement position. A thickness of the linear member 221 may have flexibility that can appropriately follow the head shape and sufficient strength that does not break even in a case where the brain wave measuring device 210 is mounted on the head 99 to measure the brain waves.

The connectors 225 at both ends of the linear member 221 are attached to the connector 265 of the electrode attachment portion 260, thereby connecting the adjacent electrode attachment portions 260 (that is, the electrode units 30).

By using the electrical wire cable as the linear member 221, the acquired brain wave signal can be collected to a predetermined external output terminal (not shown). In addition, by using two linear members 221 parallel to each other between the adjacent electrode units 30, the posture of the electrode units 30 in contact with the head 99 can be stabilized. In addition, since the linear member 221 is detachable from the electrode attachment portion 260, it is possible to replace the linear member 221 or the electrode attachment portion 260 in a case where a part of the linear member 221 or the electrode attachment portion 260 is damaged or the like. In addition, by preparing linear members 221 having different lengths, an inter-electrode distance, that is, an optimum electrode position corresponding to the size of the head 99 of the subject can be set. The support member 220 may be fixed to the electrode attachment portion 260 by soldering or the like.

The embodiments of the present invention have been described above, but these are examples of the present invention and various configurations other than the above can be adopted.

EXAMPLES

Hereinafter, the present embodiments will be described in detail with reference to Examples. The present embodiments are not limited to the description of Examples.

The following examples correspond to the first and second embodiments, and "Verification of support member (film base material)" and "Verification of modeling based on capstan equation" are confirmed.

<<Verification of support member (film base material)>>

Seven samples of Examples 1 to 7 were evaluated as to whether or not they were appropriate as the support member 20.

The evaluation standards were in three stages as follows.

Evaluation A ... deviation of the electrode position was small (5 mm or less) in a case where a certain tension (0.6 N) was applied.

Evaluation B ... deviation of the electrode position was within an allowable range (10 mm or less) in a case where a certain tension (0.6 N) was applied.

Evaluation C ... deviation of the electrode position was out of an allowable range (more than 10 mm) in a case where a certain tension (0.6 N) was applied.

Materials of each sample of Examples 1 to 7 are as follows. In addition, Table 1 shows each physical property (Poisson's ratio, Young's modulus, maximum thickness, Young's modulus $\times$ thickness).

Example 1 ... polyethylene terephthalate (PET) resin
Example 2 ... polyimide (PI) resin
Example 3 ... aluminum foil
Example 4 ... copper foil
Example 5 ... polystyrene (PS) resin
Example 6 ... polyethylene (PE) resin
Example 7 ... glass film

In Examples 1 to 6, the deviation of the electrode position in a case where a certain tension was applied was small, and thus the brain wave acquisition could be stably performed at the target position.

In Example 7, the Young's modulus of the support member was smaller than that of Examples 1 to 6, and the thickness was increased as compared with other samples in order to suppress deformation, but the deviation of the electrode in a case where a certain tension was applied was slightly large.

[Table 1]

| | Material | Poisson' s ratio | Young's modulus [GPa] | Maximum thickness [mm] | Young's modulus × thickness [GPa × mm] | Evaluation |
|---|---|---|---|---|---|---|
| Example 1 | PET | 0.25 to 0.4 | 5 | 0.5 | 2.5 | A |
| Example 2 | PI | 0.29 to 0.30 | 3.4 | 0.225 | 0.765 | A |
| Example 3 | Aluminum foil | 0.345 | 70.3 | 0.05 | 3.515 | A |
| Example 4 | Copper foil | 0.343 | 129.8 | 0.07 | 9.086 | A |
| Example 5 | PS | 0.34 to 0.38 | 2.7 to 4.2 | 0.2 | 1.08 to 2.1 | A |
| Example 6 | Glass film | 0.2 | 73 | 0.03 | 2.19 | A |
| Example 7 | PE | 0.26 | 0.4 to 1.3 | 1 | 0.4 to 1.3 | B |

<<Verification of modeling based on capstan equation>>

The brain wave measuring device described in the embodiment was mounted on a head model, an electrode pressing force F was measured using a pressure sensor disposed on the surface of the head model, and the measured value was compared with a calculated value obtained by calculation from the model based on the capstan equation.

Specific specifications of the brain wave measuring device 10 are as follows.

Brain wave measurement positions: five locations of T3, C3, Cz, C4, and T4

Electrode unit: base portion diameter of 10 mm

Thickness of base portion: 5 mm

Height of protrusion portion: 5 mm

Number of protrusion portions: 7

Distance between electrodes: 70 mm

Support member (film base material):

PI resin
Poisson's ratio: 0.3
Young's modulus: 3.4 GPa
Maximum thickness: 0.225 mm
Instead of fixing the ear to the ear attachment part, weights of 200 g were attached to both ends to simulate the mounting state of the brain wave measuring device.

FIG. 14 shows a graph of theoretical values (calculated values) and measured values of the electrode pressing force F at the five locations of T3, C3, Cz, C4, and T4. As can be seen from the drawing, it can be confirmed that the theoretical value and the measured value substantially match, and thus the modeling is appropriate.

Priority is claimed on Japanese Patent Application No. 2023-077092, filed on May 9, 2023, the disclosure of which is incorporated herein by reference.

REFERENCE SIGNS LIST

10, 110, 210 brain wave measuring device
20 support member (film base material)
25 female snap button
30 electrode unit
31 base portion
32 protrusion portion
33 conductive contact portion
34 signal line portion
35 male snap button
40 ear attachment part
50, 150 adjustment part
70 attachment part
135 male screw connection terminal
160, 260 electrode attachment portion
161, 261 attachment portion main body
162, 262 female screw
220 support member
221 linear member
225, 265 connector

## Claims

**1.** A brain wave measuring device comprising:

a support member which is disposed along a head of a subject;
an electrode unit which is attached to the support member and acquires a brain wave signal by coming into contact with a scalp of the subject; and
an assist member which assists in positioning the support member on the head,
wherein the support member is composed of a ribbon-shaped member or a line-shaped member.

**2.** The brain wave measuring device according to Claim 1,
wherein the support member is composed of a non-stretchable member which has flexibility of being deformable to follow a shape of the head.

**3.** The brain wave measuring device according to Claim 1 or 2,
wherein, in a case where the support member is composed of the ribbon-shaped member, the support member has a film member which is stretched between adjacent electrode units.

**4.** The brain wave measuring device according to Claim 3,
wherein a Poisson's ratio of a material constituting the film member is 0.15 to 0.4.

**5.** The brain wave measuring device according to Claim 3 or 4,
wherein a product of an elastic modulus and a thickness of a material constituting the film member is 0.4 to 9.1 GPa·mm.

**6.** The brain wave measuring device according to Claim 1 or 2,
wherein, in a case where the support member is composed of the line-shaped member, the line-shaped member is stretched between electrode units.

**7.** The brain wave measuring device according to Claim 6,
wherein at least two members spaced from each other are provided to extend as the line-shaped member, and the electrode units are provided between the two members.

**8.** The brain wave measuring device according to any one of Claims 1 to 7, wherein the electrode unit has a base portion, a plurality of protruding portions provided on the base portion, and an electrode portion provided on the protruding portions and in contact with the head.

**9.** The brain wave measuring device according to Claim 8, wherein the protruding portion is an elastic member.

**10.** The brain wave measuring device according to any one of Claims 1 to 9, wherein the assist member is provided at an end portion of the support member, is attached to an ear or a jaw, and assists in positioning the support member along a shape of the head.

**11.** A brain wave measuring method comprising:

attaching the brain wave measuring device according to any one of Claims 1 to 10 to a head of a subject; and measuring brain waves.

FIG. 1

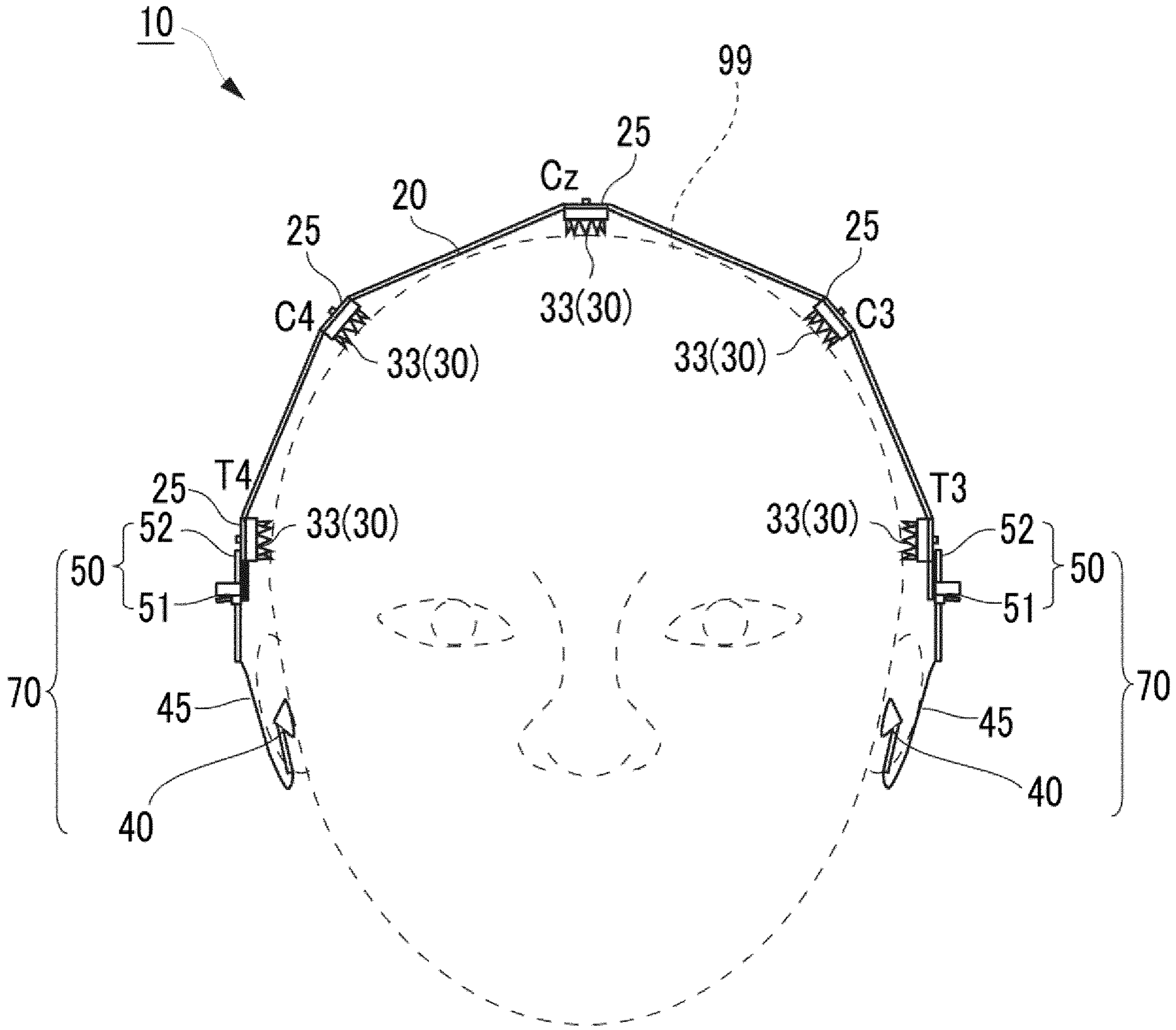
10
99
25
Cz
20
25
25
C4
33(30)
C3
33(30)
33(30)
T4
T3
25
33(30)
33(30)
52
52
50
50
51
51
70
70
45
45
40
40

FIG. 2

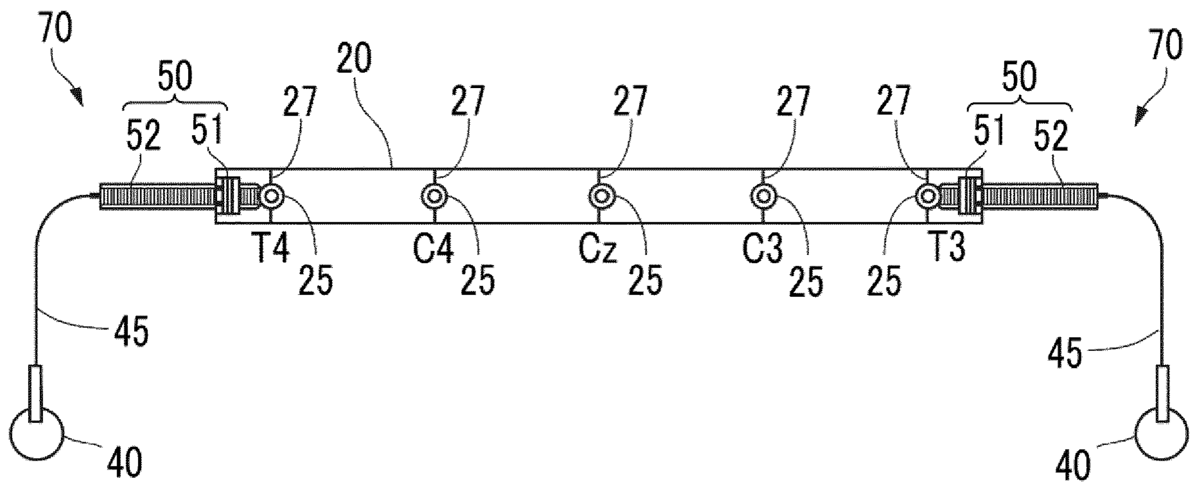
70
70
50
20
50
52
51
27
27
27
27
27
51
52
T4
C4
Cz
C3
T3
25
25
25
25
25
45
45
40
40

FIG. 3

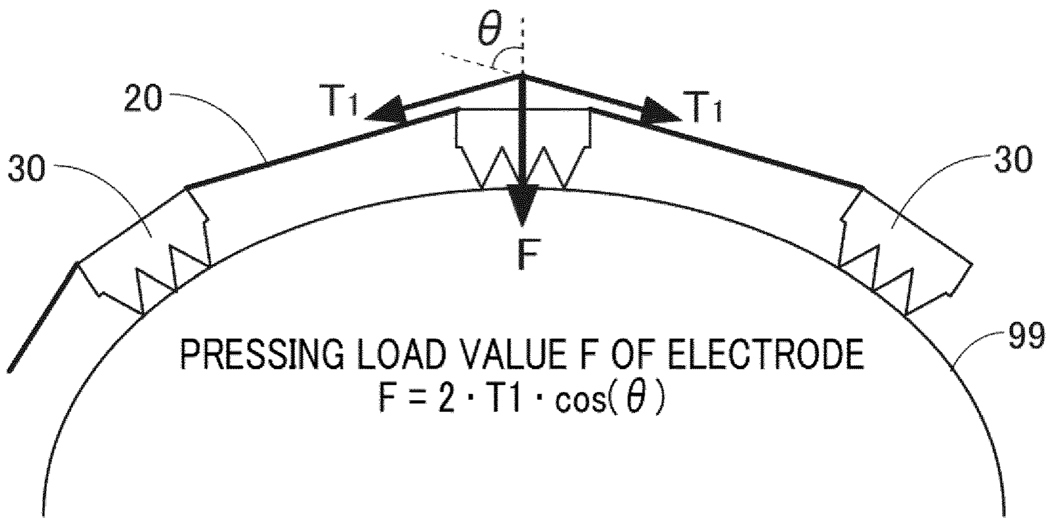


FIG. 4

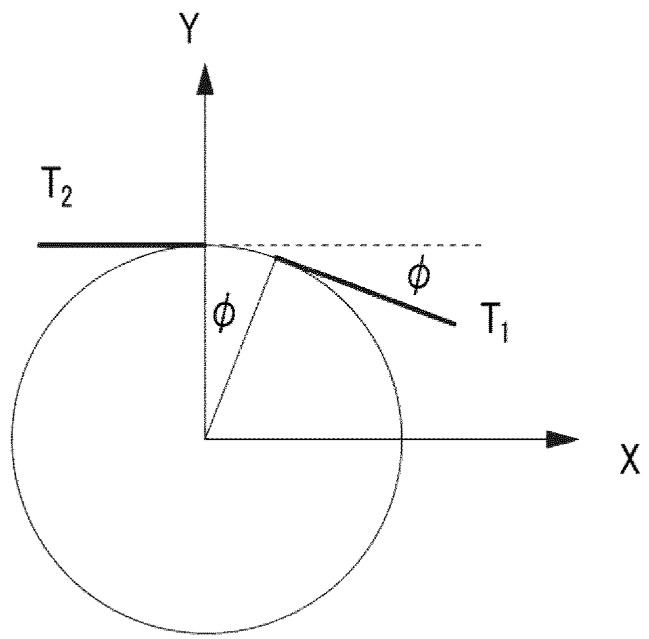


$T_1 = T_2 \cdot e^{\mu \cdot \phi}$

$\mu$: FRICTION COEFFICIENT,
$\phi$: CENTRAL ANGLE

FIG. 5

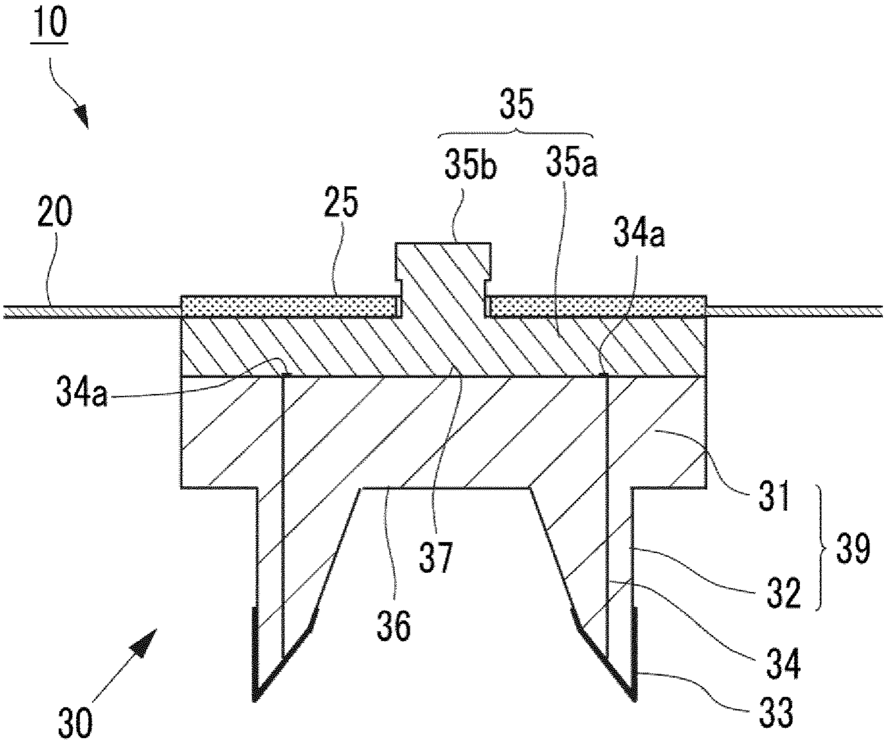

FIG. 6

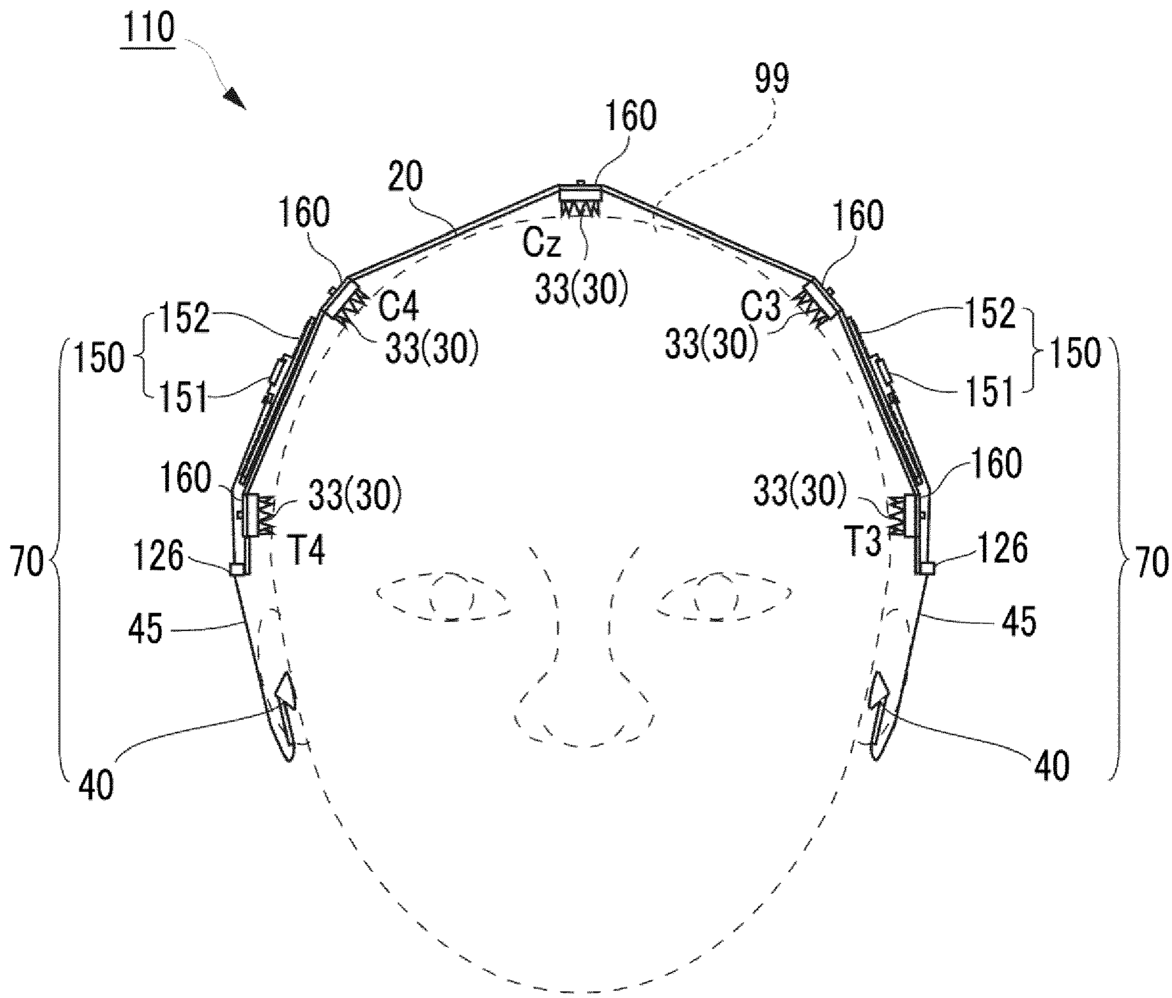
110
99
160
20
160
160
Cz
33(30)
C4
33(30)
C3
33(30)
152
150
151
152
150
151
160
33(30)
33(30)
160
T4
T3
70
126
126
70
45
45
40
40

FIG. 7

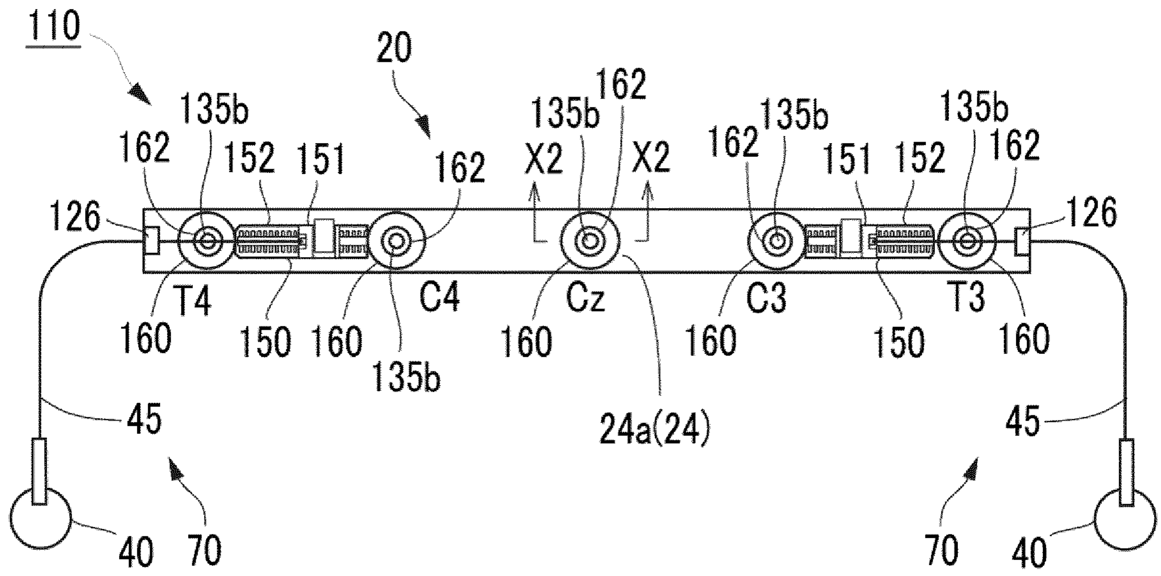
110
20
135b
162
152
151
162
135b
162
X2
X2
162
135b
151
152
135b
162
126
126
T4
C4
Cz
C3
T3
160
150
160
160
160
150
160
135b
24a(24)
45
45
40
70
70
40

FIG. 8

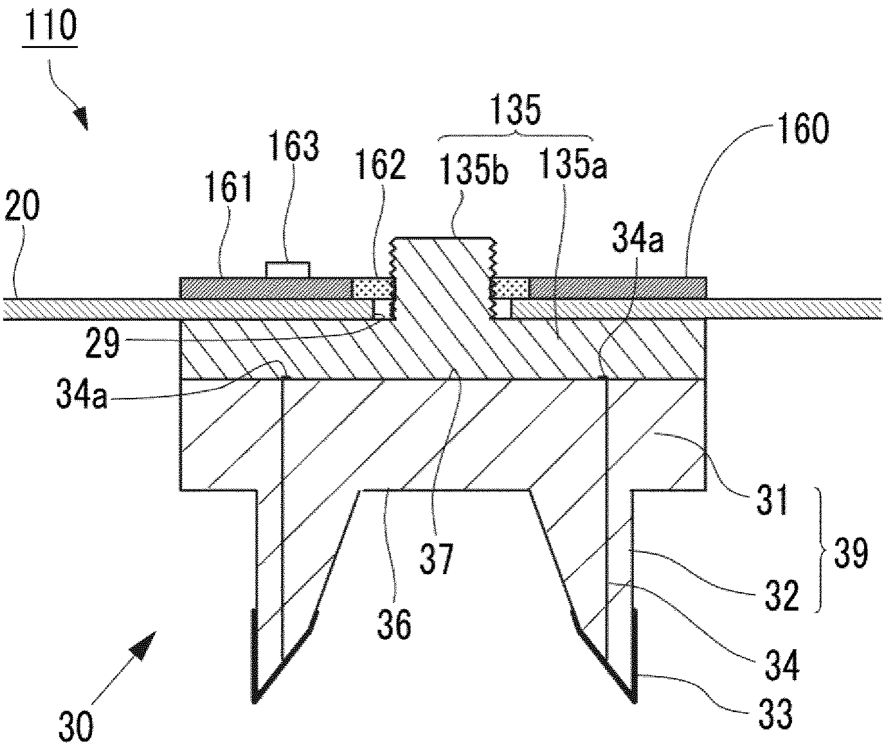


FIG. 9A

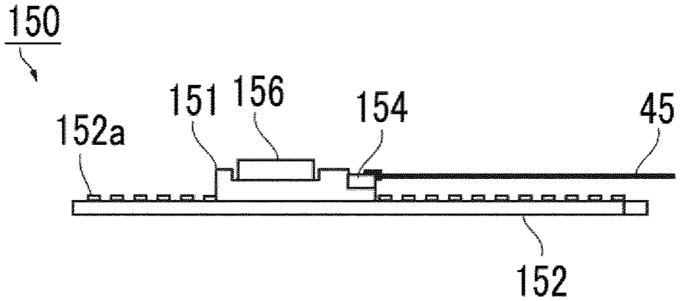


FIG. 9B

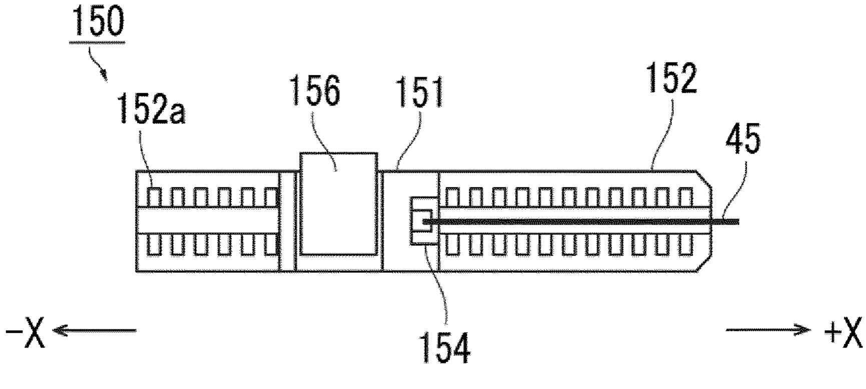

FIG. 10

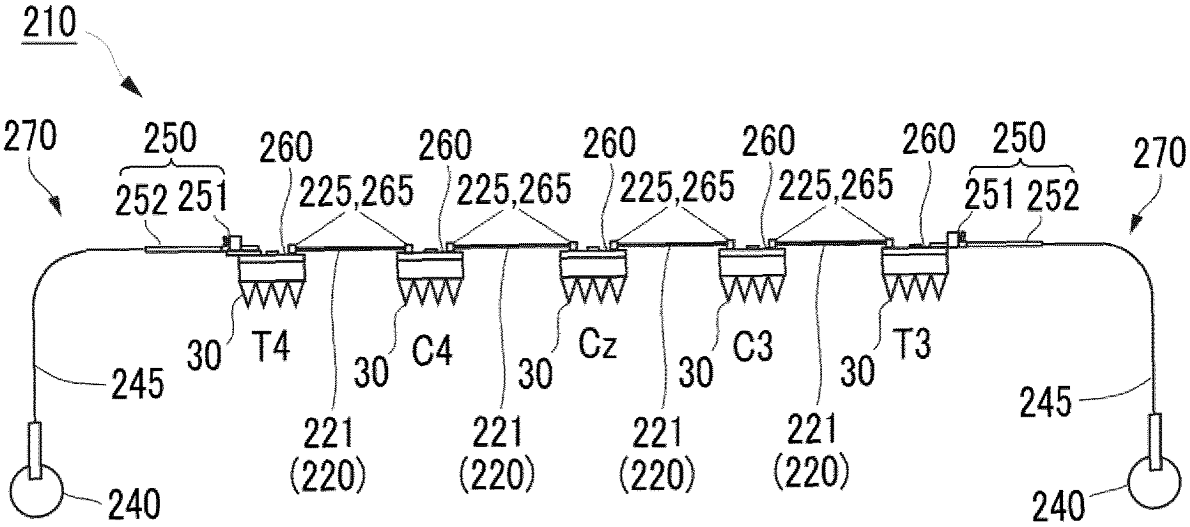
210
270
250
252 251
260
225,265
30
T4
C4
Cz
C3
T3
245
221
(220)
240

FIG. 11

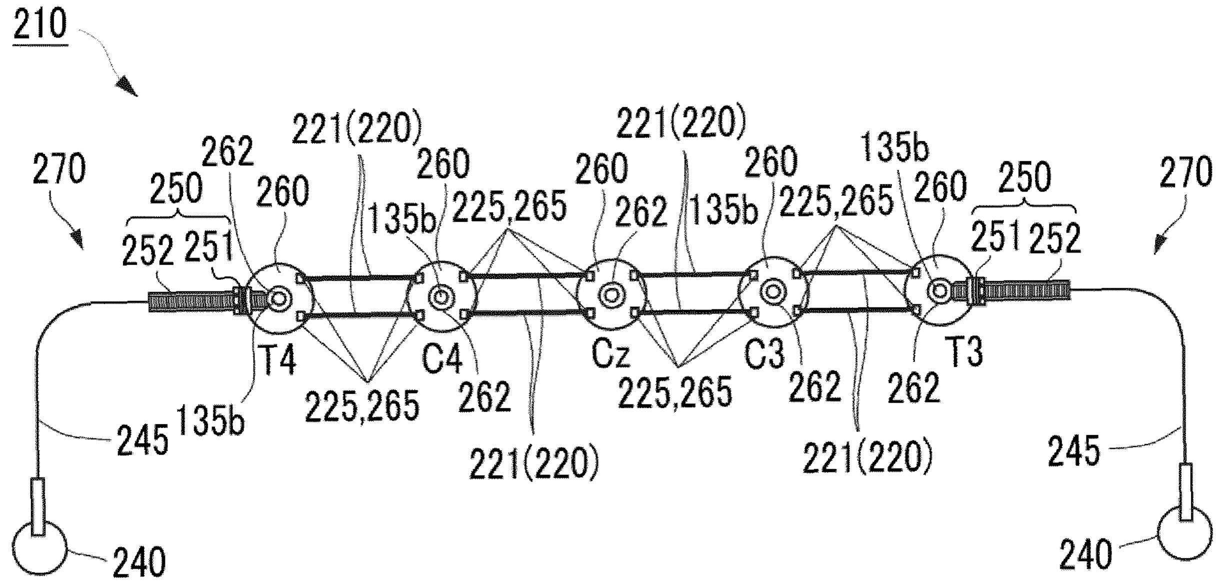
210
270
250
252 251
262
260
221(220)
135b
225,265
T4
C4
Cz
C3
T3
245
240

FIG. 12

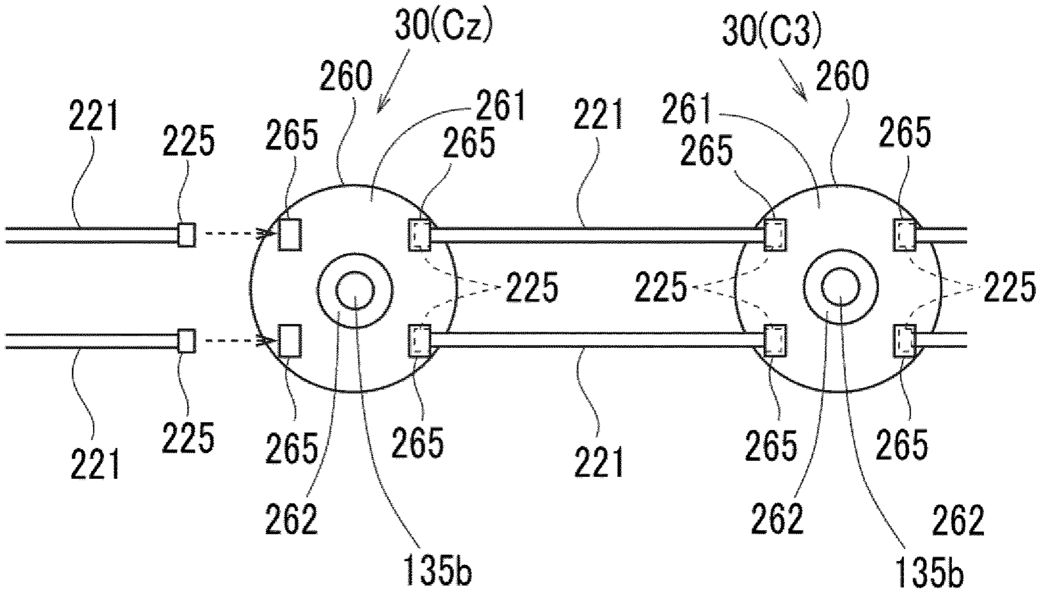
30(Cz)
30(C3)
260
261
265
225
221
262
135b

FIG. 13

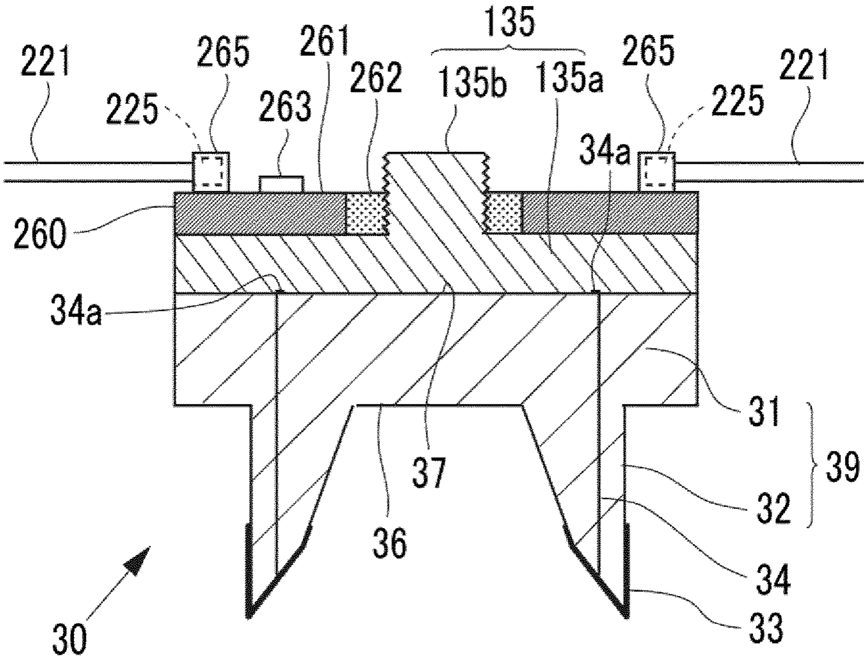
135
221
265
261
262
135b
135a
225
263
34a
260
31
39
32
37
36
34
33
30

FIG. 14

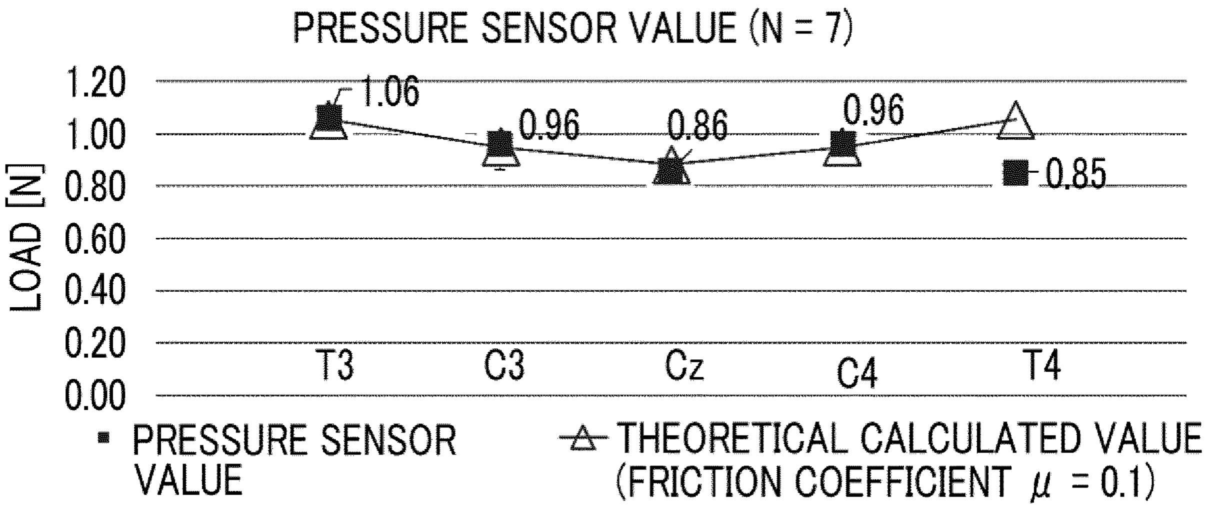
PRESSURE SENSOR VALUE (N = 7)
LOAD [N]
1.20
1.00
0.80
0.60
0.40
0.20
0.00
1.06
0.96
0.86
0.96
0.85
T3
C3
Cz
C4
T4
PRESSURE SENSOR VALUE
THEORETICAL CALCULATED VALUE (FRICTION COEFFICIENT μ = 0.1)

## INTERNATIONAL SEARCH REPORT

International application No.
**PCT/JP2024/014242**

**A. CLASSIFICATION OF SUBJECT MATTER**

***A61B 5/256***(2021.01)i; ***A61B 5/291***(2021.01)i
FI: A61B5/256 110; A61B5/291

According to International Patent Classification (IPC) or to both national classification and IPC

**B. FIELDS SEARCHED**

Minimum documentation searched (classification system followed by classification symbols)

A61B5/25-5/398

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2022-23458 A (SUMITOMO BAKELITE CO., LTD.) 08 February 2022 (2022-02-08) paragraphs [0010]-[0019], fig. 1-3 | 1-5, 8-11 |
| A | paragraphs [0010]-[0156], fig. 1-9 | 6-7 |
| Y | WO 2022/259831 A1 (SUMITOMO BAKELITE CO., LTD.) 15 December 2022 (2022-12-15) paragraphs [0012]-[0014], fig. 1-2 | 1-11 |
| Y | JP 2022-13591 A (SUMITOMO BAKELITE CO., LTD.) 18 January 2022 (2022-01-18) paragraphs [0009]-[0021], fig. 1-3 | 1-2, 6-11 |
| A | paragraphs [0009]-[0077], fig. 1-14 | 3-5 |
| A | US 2017/0055903 A1 (STICHTING IMEC) 02 March 2017 (2017-03-02) paragraphs [0034]-[0046], fig. 1-14 | 1-11 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 June 2024** | **18 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/014242**

| Patent document cited in search report | Publication date (day/month/year) | Patent family member(s) | Publication date (day/month/year) |
|---|---|---|---|
| JP 2022-23458 A | 08 February 2022 | (Family: none) | |
| WO 2022/259831 A1 | 15 December 2022 | JP 2024-20664 A paragraphs [0012]-[0014], fig. 1-2 | |
| JP 2022-13591 A | 18 January 2022 | WO 2022/004408 A1 paragraphs [0009]-[0021], fig. 1-3 | |
| US 2017/0055903 A1 | 02 March 2017 | EP 3135191 A1 paragraphs [0020]-[0033], fig. 1-14 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5900167 B
- JP 2023077092 A